(19) **Europäisches Patentamt**
**European Patent Office**
**Office européen des brevets**

(11) **EP 3 450 534 A1**

(12) **EUROPÄISCHE PATENTANMELDUNG**

(43) Veröffentlichungstag:
**06.03.2019 Patentblatt 2019/10**

(51) Int Cl.:
*C12C 5/02* *(2006.01)*          *C12C 12/00* *(2006.01)*
*A23L 31/15* *(2016.01)*

(21) Anmeldenummer: **18190459.0**

(22) Anmeldetag: **23.08.2018**

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**KH MA MD TN**

(30) Priorität: **04.09.2017   DE 102017120283**

(71) Anmelder: **Erdinger Weißbräu Werner Brombach GmbH & Co. KG**
**85435 Erding (DE)**

(72) Erfinder:
• **Fischer, Malaika**
**85435 Erding (DE)**
• **Liebert, Peter**
**85435 Erding (DE)**
• **Kreisz, Stefan**
**85435 Erding (DE)**

(74) Vertreter: **Kuhnen & Wacker**
**Patent- und Rechtsanwaltsbüro PartG mbB**
**Prinz-Ludwig-Straße 40A**
**85354 Freising (DE)**

(54) **VERFAHREN ZUR HERSTELLUNG EINES NAHRUNGSMITTELS ODER EINER VORSTUFE DESSELBEN, NAHRUNGSMITTEL ODER EINE VORSTUFE DESSELBEN UND EINE ENTSPRECHENDE VERWENDUNG**

(57)     Beansprucht wird ein Verfahren zur Herstellung eines Nahrungsmittels oder einer Vorstufe desselben, mit den Schritten: (a) Bereitstellen einer Maische oder einer Würze oder eines Glattwassers als ein erstes Nährmedium; und (b) Behandeln des ersten Nährmediums mit Milchsäurebakterien der Art *Lactobacillus rossiae* (DSM 15814$^T$) oder mit Milchsäurebakterien wenigstens umfassend zwei Arten, darunter *Lactobacillus rossiae* (DSM 15814$^T$). Ferner werden ein entsprechendes Nahrungsmittel und entsprechende Verwendungen beansprucht.

**EP 3 450 534 A1**

**Beschreibung**

Die vorliegende Erfindung betrifft ein Verfahren zur Herstellung eines Nahrungsmittels oder einer Vorstufe desselben nach Anspruch 1, ein Nahrungsmittel oder einer Vorstufe desselben nach Anspruch 11 sowie eine Verwendung nach den Anspruch 12.

Vitamin $B_{12}$ spielt bei der Zellteilung, Blutbildung, Funktion des Nervensystems, bei mentalen Fähigkeiten sowie bei der Verstoffwechselung von Kohlenhydraten, Fetten und Eiweißen eine wesentliche Rolle. Bei einem Mangel an Vitamin $B_{12}$ können daher erhebliche Stoffwechselstörungen oder sonstigen körperlichen Beeinträchtigungen, wie z.B. ein verminderter Energiestoffwechsel oder ein beeinträchtigtes Immunsystem, auftreten.

Eine ausreichende Versorgung des menschlichen oder tierischen Körpers mit Vitamin $B_{12}$ stellt daher eine wichtige Voraussetzung für Gesundheit und Leistungsfähigkeit dar.

<u>Definitionen</u>

Unter dem Begriff "Nahrungsmittel" bzw. "Lebensmittel" werden erfindungsgemäß alle Stoffe und Erzeugnisse verstanden, die der Fachmann hierunter versteht. So kann "Nahrungsmittel" alle Stoffe und Erzeugnisse umfassen, die für den menschlichen und/oder tierischen Verzehr geeignet sind und vorzugsweise eine Nährwirkung aufweisen. Insbesondere kann der Begriff "Nahrungsmittel" im Rahmen dieser Erfindung umfassen: ein getreidehaltiges Nahrungsmittel, einen getreidehaltigen Riegel, ein Malzextraktprodukt, Frühstückscerealien, eine Backware, ein Milchprodukt, ein Joghurt, ein Getränk, ein alkoholfreies Getränk, ein Bier, ein alkoholfreies Bier, ein Weizenbier, ein alkoholfreies Weizenbier, ein Biermischgetränk, ein mit einer Hefe fermentiertes Getränk, ein nicht mit Hefe fermentiertes Getränk und/oder Konzentrate der vorstehenden Nahrungsmittel. Eine Vorstufe des erfindungsgemäßen Nahrungsmittels, insbesondere des Getränks, kann insbesondere ein Sauergut, eine Maische und eine Würze sein. Im Rahmen dieser Anmeldung umfasst der Begriff "nicht fluid" in Zusammenhang mit Nahrungsmittel eine feste, pastöse, pastenartige, gelartige oder ähnliche Konsistenz eines Nahrungsmittels.

Vitamin $B_{12}$ ist ein Sammelbegriff für eine Reihe wasserlöslicher, strukturell ähnlicher Verbindungen mit biologischer Wirkung, die sog. Corrinoide. Aufgrund des komplex gebundenen Cobalt-Atoms werden sie als Cobalamine bezeichnet. Zu den biologisch aktiven Vitamin $B_{12}$-Formen zählt der Fachmann: Methylcobalamin, Desoxyadenosylcobalamin, Hydroxycobalamin und Sulfitcobalamin. Von diesen werden Methylcobalamin und Desoxyadenosylcobalamin als die biologisch wirksamsten bzw. aktivsten Formen angesehen. Von dieser Gruppe ist Cyanocobalamin nur auf künstlichem, d.h. synthetischem Wege zugänglich.

Neben diesen aktiven Vitamin $B_{12}$-Formen gibt es auch sogenannte inaktive Analoga. Diese werden auch Pseudo-Vitamin $B_{12}$ genannt, weil sie zwar eine ähnliche chemische Struktur wie das echte Vitamin $B_{12}$ aufweisen, aber unter Umständen keine Vitamin-Wirkung für den menschlichen oder tierischen Körper entfalten. Sie sind nicht geeignet, physiologische Aufgaben des Vitamins $B_{12}$ im Organismus zu erfüllen. Weiterhin können sie die Aufnahme und Verstoffwechselung von biologisch aktivem Vitamin $B_{12}$ blockieren.

Unter Pseudo-Vitamin $B_{12}$ wird im Rahmen dieser Anmeldung insbesondere 7-Adeninyl-Cyanocobamide verstanden.

Im Rahmen dieser Anmeldung wird die Definition des Begriffs "Vitamin $B_{12}$" auf die im menschlichen oder tierischen Organismus aktiven Formen des Vitamins $B_{12}$ oder deren Vorstufen beschränkt. Darüber hinaus ist der Begriff "Vitamin $B_{12}$" im Rahmen dieser Anmeldung auf Formen des Vitamin $B_{12}$ beschränkt, welche sich mikrobiologisch erzeugen lassen. Da sich das vorstehend erwähnte Cyanocobalamin nur auf synthetischem Wege erzeugen lässt und daher kein "natürliches" Vitamin $B_{12}$ darstellt, ist diese Form des Vitamin $B_{12}$ von dem Begriff "Vitamin $B_{12}$" im Rahmen dieser Anmeldung nicht umfasst.

Damit beschränkt sich die Definition des Begriffs "Vitamin $B_{12}$" im Rahmen dieser Anmeldung auf die nachfolgenden Vitamin $B_{12}$-Spezies: Methylcobalamin, Desoxyadenosylcobalamin, Hydroxycobalamin und Sulfitcobalamin.

Die Vertreter der vorstehend genannten Gruppe zeichnen sich im Allgemeinen durch eine hohe Bioverfügbarkeit aus, so dass diese geeignet sind, die Versorgung des menschlichen oder tierischen Körpers mit Vitamin $B_{12}$ zu verbessern.

Im Rahmen dieser Anmeldung wird der Begriff "Bioverfügbarkeit" als die Resorption des Nährstoffs (Vitamin $B_{12}$) vom Gastrointestinaltrakt ins Blut verstanden, wodurch dieser in den systemischen Kreislauf gelangt und somit den Zellen/Organen zur Verfügung steht. Des Weiteren wird auf die Definition des Begriffs "bioverfügbar" oder "Bioverfügbarkeit" auf Absatz [0013] der Beschreibung der Internationalen Patent-Offenlegungsschrift WO 2012/109 324 A1 verwiesen. Auf letztgenanntes Dokument wird hiermit vollinhaltlich Bezug genommen.

Zur Bestimmung von bioverfügbarem Vitamin $B_{12}$ wurde eine Nachweisanalytik auf Basis des sog. ADVIA-Systems eingesetzt. Dieser Bioverfügbarkeitsnachweis basiert auf dem sogenannten Intrinsic Factor (IF) und wird bspw. zum Nachweis von bioverfügbarem Vitamin $B_{12}$ im Blut verwendet. Vitamin $B_{12}$ wird im letzten Dünndarmabschnitt (Ileum) mittels des Intrinsic Factors (IF = Protein, welches in den Parietalzellen der Magenschleimhaut sezerniert wird) absorbiert. Im Rahmen dieses Tests wird das IF-ungebundene Vitamin $B_{12}$ als nicht bioverfügbar definiert, da dieses nicht in den Blutkreislauf und hiermit einhergehend nicht an das entsprechende Gewebe gelangt.

In der vorliegenden Anmeldung wurde der ADVIA Centaur VB12-Test (111659 Rev. N, 2008-09; VB12 2/12) zum Nachweis von bioverfügbarem Vitamin $B_{12}$ verwendet. Dieser ist ein kompetitiver Immunoassay unter Anwendung der direkten Chemilumineszenz-Technologie. Dabei konkurriert Vitamin $B_{12}$ in der Probe mit dem mit Acridiniumester markierten Vitamin $B_{12}$ in einem Lite-Reagenz um eine begrenzte Menge an gereinigtem IF, der kovalent an paramagnetische Partikel in der Solid Phase gebunden ist. In diesem Test werden das Releasing Agens (Natriumhydroxid) und DTT zur Freisetzung von Vitamin $B_{12}$ aus endogenen Bindungsproteinen in der Probe verwendet. Zugesetztes Cobinamid verhindert eine Rückbindung nach der Zugabe der Solid Phase zur Probe.

Zur Bestimmung der (Gesamt-)Konzentration an Vitamin $B_{12}$, welche alle Formen des Vitamins $B_{12}$, also bioverfügbare und nicht-bioverfügbare Formen, erfasst, wird im Rahmen dieser Anmeldung die Messmethode r-Biopharm AOAC-Methode Nr. 101002 verwendet. Alternativ kann zur Bestimmung der Gesamt-Konzentration die Methode "Fresenius AOAC-Methode Nr. 952.20" verwendet werden.

In Zusammenhang mit einem Hefeerzeugnis wird erfindungsgemäß unter dem Begriff "Autolysat" ein meist flüssiges Nährsubstrat verstanden, das durch Auflösen der Zellen von Backhefe, Futterhefe (Eiweißhefe) und/oder insbesondere von Bierhefe durch zelleigene Enzyme gewonnen wird.

In Zusammenhang mit einem Hefeerzeugnis wird erfindungsgemäß unter dem Begriff "Extrakt" ein meist pulver-, gel- oder pastenartiges Erzeugnis aus Hefeautolysat (mittels eigenen Hefeenzymen erzeugt) oder Hefehydrolysat (mittels Fremdenzymen erzeugt) mit einem hohen Gehalt pro Trockenmasse an Aminosäuren (bspw. 30 bis 50 %), Kohlenhydraten (bspw. 20 bis 30 %) und Vitaminen (insbesondere: B-Gruppe: Thiamin, Riboflavin, Nicotinsäure) verstanden. Allgemein wird ein Hefeextrakt hergestellt, indem ein Hefeautolysat oder -hydrolysat von den unlöslichen Zellbestandteilen wenigstens teilweise befreit, aufkonzentriert und gegebenenfalls sprühgetrocknet wird. Typischerweise weist ein Hefeextrakt einen Trockensubstanzgehalt von 70 bis 80 % bei pastenartiger Konsistenz und von 95 bis 97 % bei Pulverkonsistenz auf.

In Zusammenhang mit einem Hefeerzeugnis wird erfindungsgemäß unter dem Begriff "getrocknete Gestalt" jede Gestalt einer Hefe verstanden, welche einen Wassergehalt von höchstens 5 %, vorzugsweise höchstens 2 %, insbesondere höchstens 1 %, aufweist. Insbesondere wird hierunter eine getrocknete Hefe in Gestalt von Flocken oder Pulver oder eine gefriergetrocknete Hefe verstanden.

Dem Begriff "Sauergut" wird im Rahmen dieser Erfindung die übliche, im Gebiet der Brauereitechnologie geläufige Definition zugeordnet. Hierunter wird insbesondere ein zur pH-Absenkung mikrobiell, vorzugsweise mit Milchsäurebakterien, behandeltes Nährmedium, insbesondere eine Maische und/oder Würze, verstanden.

Im Rahmen dieser Anmeldung wird unter den Begriffen "Maische", "Würze" und "Glattwasser" insbesondere die Substrate verstanden, welche der Fachmann unter derselben Bezeichnung aus dem Bierbereitungsprozeß kennt. Die Begriffe sind aber erfindungsgemäß nicht notwendigerweise hierauf beschränkt, sondern können sich auch auf analoge Substrate, also Vorstufen, hinsichtlich sonstiger Getränke oder anderer Nahrungsmittel, wie beispielsweise Whiskey-Maischen, beziehen. Insbesondere kann erfindungsgemäß die Würze eine aus einer erfindungsgemäßen Maische, wie hierin definiert, hergestellt werden.

Vorzugsweise kann der Begriff "Maische" erfindungsgemäß auf eine Maische beschränkt sein, welche unter Verwendung eines kohlenhydrathaltigen Substrats oder eines Gemisches von kohlenhydrathaltigen Substraten hergestellt wurde. Dabei weist das kohlenhydrathaltige Substrat oder das Gemisch von kohlenhydrathaltigen Substraten einen Anteil an Braumalz von wenigstens 80 Massen-%, vorzugsweise wenigstens 90 Massen-%, vorzugsweise wenigstens 95 Massen-%, vorzugsweise wenigstens 98 Massen-%, vorzugsweise wenigstens 99 Massen-%, insbesondere etwa 100 Massen-%, auf.

Insbesondere kann der Begriff "Maische" erfindungsgemäß auf eine Maische beschränkt sein, welche unter Verwendung von Braumalz hergestellt wurde, wobei das Braumalz einen Anteil an Weizenmalz von wenigstens 50 Massen-%, vorzugsweise wenigstens 52 Massen-%, insbesondere wenigstens 55 Massen-%, aufweist. Erfindungsgemäß schließt der Begriff "Braumalz" Malze einer Sorte und auch Mischungen verschiedener Malzsorten ein.

Ferner kann der Begriff "Würze" erfindungsgemäß auf eine Würze beschränkt sein, welche aus einer Maische gewonnen wurde, welche unter Verwendung von Braumalz mit einem Anteil an Weizenmalz von wenigstens 50 Massen-%, vorzugsweise wenigstens 52 Massen-%, insbesondere wenigstens 55 Massen-%, hergestellt wurde. Erfindungsgemäß kann die Würze eine Würze sein, welche aus der Maische wie vorstehend beschrieben hergestellt wurde.

Unter dem Begriff "Hopfenbitterstoffe" werden im Rahmen dieser Anmeldung alle dem Fachmann bekannten Bitterstoffe des Hopfens und Hopfenharze verstanden. Hierzu gehören sowohl die Weichharze als auch die Hartharze, einschließlich der Bittersäuren, insbesondere der α-Säuren und β-Säuren, und die bekannten Derivate dieser Harze und Säuren, insbesondere deren Oxidationsprodukte.

Unter dem Begriff "frei von Hopfenbitterstoffen" in Bezug auf ein Medium (z.B. Nährmedium oder Hefeerzeugnis) wird im Rahmen dieser Anmeldung die vollständige Abwesenheit von Hopfenbitterstoffen in diesem Medium verstanden.

Unter dem Begriff "im Wesentlichen frei von Hopfenbitterstoffen" in Bezug auf ein erstes Nährmedium wird im Rahmen dieser Anmeldung ein Gehalt an Hopfenbitterstoffen von höchstens 15 %, vorzugsweise höchstens 10 %, vorzugsweise höchstens 5 %, insbesondere höchstens 2 %, bezogen auf den Gehalt an Hopfenbitterstoffen, den eine brauereiübliche Anstellwürze für eine Vergärung mit einer untergärigen oder obergärigen Hefe mit Bittereinheiten (EBC-Methode) im Bereich von 15 bis 38, vorzugsweise 20 bis 35, aufweist. Dabei können erfindungsgemäß die vorstehenden Prozentangaben auch auf jede Einzelsubstanz der Gruppe der Hopfenbitterstoffe (z.B. Humulon oder Lupulon) angewandt werden.

Unter dem Begriff "im Wesentlichen frei von Hopfenbitterstoffen" in Bezug auf ein Hefeerzeugnis wird im Rahmen dieser Anmeldung ein Gehalt an Hopfenbitterstoffen von höchstens 20 %, vorzugsweise höchstens 15 %, vorzugsweise höchstens 10 %, vorzugsweise höchstens 5 %, insbesondere höchstens 2 %, bezogen auf den Gehalt an Hopfenbitterstoffen, den eine untergärige oder obergärige Betriebshefe in einer Brauerei, insbesondere eine frische Erntehefe der ersten, zweiten oder dritten Führung, aufweist, die während einer Vergärung einer brauereiübliche Würze mit Bittereinheiten (EBC-Methode) im Bereich von 15 bis 38, vorzugsweise 20 bis 35, geerntet wurde. Dabei können erfindungsgemäß die vorstehenden Prozentangaben auch auf jede Einzelsubstanz der Gruppe der Hopfenbitterstoffe (z.B. Humulon oder Lupulon) angewandt werden.

In analoger Weise zu den vorstehenden Definitionen ist im Rahmen dieser Anmeldung auch der Begriff "Hopfenbitterstoffe ... vollständig oder im Wesentlichen vollständig entfernt..." bezogen auf eine Hefe oder ein Hefeerzeugnis zu verstehen.

Im Rahmen dieser Anmeldung schließt der Begriff "Bier" ein teil- oder endvergorenes Bier, ein abfüllfertiges Bier, ein filtriertes Bier, ein nicht filtriertes Bier, ein Schankbier, ein Vollbier, ein alkoholfreies oder ein teilentalkoholisiertes Bier, ein Getränk auf Würzebasis oder ein Biermischgetränk sowie eine Vorstufe derselben mit ein. Insbesondere schließt der Begriff "Bier" ein Weizenbier und ein teilweise oder vollständig entalkoholisiertes bzw. alkoholfreies Weizenbier oder mit diesen hergestellte Mischgetränke ein.

Der Gegenstand der Erfindung kann sich auf die Herstellung von alkoholischen und nicht alkoholischen Getränken in der Brauerei, insbesondere von Bier, und die entsprechenden Erzeugnisse beziehen. Im Rahmen dieser Anmeldung wird dann den Begriffen "Vorderwürze", "Kochen", "Heißhalten", "Trub", "Anstelltemperatur", "Reinzuchthefe", "Erntehefe", "Waschen der Hefe", "Verarbeiten zu einem Getränk", "Würzesäuerung", "Maischesäuerung" usw. jeweils die Bedeutung zugeordnet, die ein Fachmann im Bereich der Getränkeherstellung, insbesondere im Bereich der Bierherstellung, dem jeweiligen Fachbegriff oder der Tätigkeit üblicherweise zuordnet. Analoges gilt bei der Verwendung von Begriffen für Gegenstände oder Tätigkeiten innerhalb dieser Anmeldung, die sich auf die Herstellung oder Behandlung von festen oder pastösen bzw. gelartigen Nahrungsmitteln und die entsprechenden Erzeugnissen beziehen.

Unter "Behandlung" oder "Behandeln" mit Milchsäurebakterien oder Hefe wird im Rahmen dieser Anmeldung jede Art der teilweisen oder vollständigen Verstoffwechselung von aus einem Nährmedium stammenden Nährstoffen, insbesondere eine teilweise oder vollständige Vergärung bzw. Fermentation, durch Milchsäurebakterien, verstanden. Darüber hinaus kann "Behandeln" jede Art des Inkontaktbringens oder Inkontaktseins von Mikroorganismen, vorzugsweise Milchsäurebakterien oder Hefe, insbesondere von den in dieser Anmeldung erfindungsgemäß vorgesehenen Milchsäurebakterien und Bierhefen, mit einem Nährmedium, sein.

Der Begriff "Milchsäurebakterien" im Sinne dieser Anmeldung umfasst jede Art und Zustand von Milchsäurebakterien, d.h. jede beliebige Art bzw. Unterart bzw. jeden beliebigen Stamm, soweit dies in dieser Anmeldung nicht weiter beschränkt ist. Ferner umfasst dieser Begriff lebende und/oder tote Bakterienzellen, insbesondere lebende Bakterienzellen. Die in dieser Anmeldung angegebenen DSMZ-Nummern dienen lediglich einer eindeutigen Identifizierung der erfindungsgemäß verwendeten Bakterienarten oder -unterarten. Die Erfindung ist jedoch nicht auf die DSMZ als alleinige Bezugsquelle für diese erfindungsgemäß verwendeten Bakterienarten oder -unterarten beschränkt.

Erfindungsgemäß wird unter "Weizenbier" ein obergäriges Bier mit einem Weizenmalzanteil von wenigstens 50 Massen-%, vorzugsweise wenigstens 52 Massen-%, insbesondere wenigstens 55 Massen-%, in der Schüttung oder im kohlenhydrathaltigen Substrat verstanden.

Unter dem Begriff "alkoholfrei" wird in Bezug auf ein Nahrungsmittel, Getränk oder Bier oder ein sonstiges in dieser Anmeldung genanntes Erzeugnis erfindungsgemäß ein Gehalt an Ethanol im Erzeugnis von 0 bis weniger als 1,2 Volumen-%, vorzugsweise von 0 bis weniger als 1,0 Volumen-%, vorzugsweise von 0 bis weniger als 0,5 Volumen-%, vorzugsweise von 0 bis weniger als 0,3 Volumen-%, vorzugsweise von 0 bis weniger als 0,1 Volumen-%, insbesondere etwa 0 Volumen-%, verstanden.

Im Rahmen dieser Anmeldung wird unter den Angaben "etwa", "ungefähr" oder ähnlichem eine relative Abweichung vom jeweiligen Bezugswert von höchstens 10 %, vorzugsweise höchstens 5 %, vorzugsweise höchstens 3 %, insbesondere höchstens 1 %, verstanden.

Die in dieser Anmeldung verwendeten Volumen- oder Volumenanteils-Angaben beziehen sich immer auf die Temperatur oder Temperaturen, welche ein Fachmann dem darauf bezogenen Fluid oder Gemisch für den jeweiligen Verwendungszweck bzw. beim jeweiligen Verfahrensschritt typischerweise zuordnen würde, soweit eine Temperatur nicht explizit angegeben ist.

Alle in der Anmeldung angegebenen Massen und Massenkonzentrationen beziehen sich jeweils auf die Trockenmasse des betreffenden Stoffs.

## Stand der Technik

Im Dokument EP 824 152 B1 wird die Erzeugung von natürlichem Vitamin $B_{12}$ in relativ hohen Konzentrationen ($\geq$ 0,1 Gew.-%) unter Verwendung von *Propionibacterium freudenreichii* beschrieben.

In ähnlicher Weise betrifft GB 793 467 A die Erzeugung von Zubereitungen, welche eine hohe Vitamin $B_{12}$-Aktivität insbesondere durch Kultivierung eines bestimmten Typs von *Propionibacterium,* insbesondere *P. freudenreichii* oder *P. shermanii,* aufweisen. Hierbei wird ein Verfahren zur Erzeugung einer physiologisch aktiven Zubereitung von Vitamin $B_{12}$ offenbart, welche frei von Pseudo- oder inaktiven Vitamin $B_{12}$-Varianten ist.

Allerdings wäre es wünschenswert, wenn die Menge oder Konzentration an Vitamin $B_{12}$, welche in den Erzeugnissen, die mit den vorstehend diskutierten Verfahren erzeugt werden, enthalten ist, weiter gesteigert werden könnte.

## Aufgabe der Erfindung

Aufgabe der vorliegenden Erfindung ist es, ein verbessertes Verfahren zur Herstellung eines Nahrungsmittels oder einer Vorstufe desselben, insbesondere eines Sauerguts, einer Maische, einer Würze, eines Getränks oder eines nicht fluiden Nahrungsmittels, und das entsprechende Nahrungsmittel oder eine Vorstufe desselben bereitzustellen, wobei dieses Nahrungsmittel oder bereits eine Vorstufe davon geeignet ist, die Vitamin $B_{12}$-Versorgung des menschlichen und/oder tierischen Körpers bei Aufnahme dieses Nahrungsmittel oder einer Vorstufe desselben weiter zu verbessern.

Es ist ein Aspekt der vorliegenden Erfindung, ein Nahrungsmittel oder eine Vorstufe desselben bereitzustellen, welches oder welche eine gesteigerte Menge oder Konzentration an Vitamin $B_{12}$ enthält.

Es ist ein weiterer Aspekt der vorliegenden Erfindung, ein Nahrungsmittel oder eine Vorstufe desselben bereitzustellen, welches oder welche auf natürliche Weise hergestellt worden ist und/oder dessen Rohstoffe nach dem deutschen Reinheitsgebot zulässig sind bzw. dem deutschen Reinheitsgebot entsprechen, welche sind: Gerstenmalz, Weizenmalz, eine Mischung aus Gerstenmalz und Weizenmalz, Hopfen, aus Hopfen erzeugte Produkte und Wasser.

Es ist ein weiterer Aspekt dieser Erfindung, die vorstehend definierte Aufgabe und/oder wenigstens einen der vorstehend definierten Aspekte auf einfache technische Weise und kostengünstig, insbesondere mit den in einer Brauerei herkömmlich vorhandenen Ausstattung, zu verwirklichen.

## Zusammenfassung der Erfindung

Die erfindungsgemäße Aufgabe wird durch die Gegenstände der Ansprüche 1 bis 14 wie auch durch die nachfolgend definierten Gegenstände gelöst.

In verfahrenstechnischer Hinsicht wird die erfindungsgemäße Aufgabe durch ein Verfahren zur Herstellung eines Nahrungsmittels oder einer Vorstufe desselben gemäß Anspruch 1 gelöst. Dabei weist das Verfahren wenigstens die nachfolgenden Schritte auf:

(a) Bereitstellen einer Maische oder einer Würze oder eines Glattwassers als ein erstes Nährmedium; und
(b) Behandeln des ersten Nährmediums mit Milchsäurebakterien der Art *Lactobacillus rossiae* (DSM 15814$^T$ bzw. DSM 15814) oder mit Milchsäurebakterien wenigstens umfassend zwei Arten, darunter *Lactobacillus rossiae* (DSM 15814$^T$).

Durch die Behandlung des ersten Nährmediums mit den vorstehend genannten Milchsäurebakterien wird ein Medium, insbesondere ein Sauergut, erhalten, das Vitamin $B_{12}$ enthält. Dabei haben die Erfinder überraschenderweise festgestellt, dass gerade durch den Einsatz von Milchsäurebakterien der Art *Lactobacillus rossiae* (DSM 15814$^T$) erhöhte Gehalte an Vitamin $B_{12}$ im behandelten Medium erzielt werden können.

Erfindungsgemäß ist das erste Nährmedium eine Maische oder eine Würze, vorzugsweise eine Vorderwürze oder ein Glattwasser. Vorzugsweise weist das erste Nährmedium einen Extraktgehalt im Bereich von 4 bis 24 °P, vorzugsweise 5 bis 20 °P, vorzugsweise 10 bis 18 °P, vorzugsweise 12 bis 17 °P, insbesondere 13 bis 15 °P, auf.

Insbesondere bei der Verwendung von Maische oder Würze als erstem Nährmedium hat die Anmelderin überraschend festgestellt, daß die erfindungsgemäß eingesetzten Milchsäurebakterien in einem derartigen Milieu vermehrt Vitamin $B_{12}$ erzeugen und damit zu einer Erzeugung von Vitamin $B_{12}$ geeignet sind. Ferner wurde festgestellt, daß das mittels der erfindungsgemäß eingesetzten Milchsäurebakterien erzeugte Vitamin $B_{12}$ auch bioverfügbar in Sinne dieser Anmeldung ist. Insbesondere können im erfindungsgemäß behandelten ersten Medium Gehalte an bioverfügbarem Vitamin $B_{12}$ im Bereich vom 5 bis 15 $\mu$g/100 mL, vorzugsweise 6 bis 12 $\mu$g/100 mL, erzielt werden (gemessen mittels ADVIA Centaur VB12-Test).

Bei Anwendung des erfindungsgemäßen Verfahrens kann also eine mengenmäßig nochmals gesteigerte Erzeugung von Vitamin $B_{12}$, insbesondere bioverfügbarem Vitamin $B_{12}$, erreicht werden, offensichtlich ohne daß ein "Umschalten" des Stoffwechsels der erfindungsgemäß vorgesehenen Milchsäurebakterien auf die Erzeugung von nicht gewünschten Substanzen stattfindet. Ein derartiges Umschalten ist beispielsweise von Milchsäurebakterien anderer Arten, wie beispielweise *Lactobacillus reuterii,* bekannt.

Vorteilhafte Ausführungsformen des erfindungsgemäßen Verfahrens sind Gegenstand der abhängigen Ansprüche.

So kann das erfindungsgemäße Verfahren als Schritt (b) den Schritt aufweisen:

(b) Behandeln des ersten Nährmediums mit Milchsäurebakterien der Arten *Lactobacillus rossiae* (DSM 15814$^T$) und *Lactobacillus coryniformis,* insbesondere der Unterart *Lactobacillus coryniformis* subsp. *coryniformis* (DSMZ Nr. 20007), vorzugsweise mit einem Gemisch enthaltend Milchsäurebakterien der Arten *Lactobacillus rossiae* (DSM 15814$^T$) und *Lactobacillus coryniformis,* insbesondere der Unterart *Lactobacillus coryniformis* subsp. *coryniformis* (DSMZ Nr. 20007).

Überraschenderweise kann durch die Behandlung des ersten Nährmediums mit einer Kombination aus *Lactobacillus rossiae* und *Lactobacillus coryniformis* eine noch höhere Konzentration an Vitamin $B_{12}$ im behandelten Medium erzielt werden.

Die Erfinder haben hierzu eine Arbeitshypothese aufgestellt und vermuten, dass die erhöhte Ausbeute an Vitamin $B_{12}$ möglicherweise auf einen Synergieeffekt zurückzuführen ist, der durch das Zusammenwirken von *Lactobacillus rossiae* und *Lactobacillus coryniformis* bewirkt wird. So wird vermutet, dass die Art *Lactobacillus rossiae* Riboflavin bildet, welche scheinbar von der Art *Lactobacillus coryniformis* für die Synthese von Vitamin $B_{12}$ vorteilhaft genutzt werden kann.

Dabei kann das Volumenverhältnis der eingesetzten Milchsäurebakterien-Suspensionen (*Lactobacillus rossiae* zu *Lactobacillus coryniformis*) in Schritt (b) zwischen 10:90 bis 85:15, vorzugsweise 15:85 bis 50:50, vorzugsweise 15:85 bis 35:65, insbesondere 20:80 bis 30:70, bezogen/normiert auf die gleiche Zellzahl, betragen. Durch die Wahl der vorstehenden Volumenverhältnisse werden nach Erkenntnis der Erfinder eine hohe Konzentration an Vitamin $B_{12}$ im behandelten Medium erzielt.

In einer weiteren, vorteilhaften Ausführungsform kann die Behandlung gemäß Schritt (b) in Anwesenheit eines Hefeerzeugnisses stattfinden, wobei das Hefeerzeugnis ein Extrakt, ein Autolysat und/oder eine Hefe in frischer oder getrockneter Gestalt enthält oder aus einem Extrakt, einem Autolysat und/oder einer Hefe in frischer oder getrockneter Gestalt besteht. Erfindungsgemäß kann das Hefeerzeugnis auch ein beliebiges Gemisch aus einem Extrakt, einem Autolysat und/oder einer Hefe in frischer oder getrockneter Gestalt sein oder enthalten. In Schritt (b) kann die Massenkonzentration des Hefeerzeugnisses bezogen auf das erste Nährmedium vorzugsweise $\geq 0{,}2$ g/L, vorzugsweise $\geq 4$ g/L, vorzugsweise $\geq 8$ g/L, vorzugsweise $\geq 15$ g/L, insbesondere $\geq 18$ g/L, betragen. Zusätzlich oder alternativ kann in Schritt (b) die Massenkonzentration des Hefeerzeugnisses bezogen auf das erste Nährmedium vorzugweise $\leq 70$ g/L, vorzugweise $\leq 50$ g/L, vorzugsweise $\leq 40$ g/L, vorzugsweise $\leq 30$ g/L, insbesondere $\leq 25$ g/L, betragen. Insbesondere kann in Schritt (b) die Massenkonzentration des Hefeerzeugnisses bezogen auf das erste Nährmedium im Bereich von $\geq 0{,}2$ bis $\leq 70$ g/L, vorzugsweise von $\geq 8$ bis $\leq 50$ g/L, vorzugsweise von $\geq 12$ bis $\leq 40$ g/L, insbesondere von $\geq 18$ bis $\leq 35$ g/L, sein.

Überraschenderweise kann durch die Anwesenheit eines Hefeerzeugnisses oder einer Kombination der beschriebenen Hefeerzeugnisse während der Behandlung des ersten Nährmediums mit Milchsäurebakterien der Art *Lactobacillus rossiae* oder einem Gemisch von Milchsäurebakterien enthaltend diese Art, insbesondere mit einem Gemisch enthaltend Milchsäurebakterien der Arten *Lactobacillus rossiae* (DSM 15814$^T$) und *Lactobacillus coryniformis,* insbesondere der Unterart *Lactobacillus coryniformis* subsp. *coryniformis* (DSMZ Nr. 20007), die Menge und/oder Massenkonzentration an erzeugtem Vitamin $B_{12}$ im ersten Nährmedium gesteigert werden.

Ab einer Zugabe oder Massenkonzentration des Hefeerzeugnisses von etwa 0,2 g/L und insbesondere ab 4 g/L (bezogen auf das erste Nährmedium) kann eine Steigerung der erzeugten Menge und/oder Massenkonzentration an Vitamin $B_{12}$ beobachtet werden, auch wenn das Hefeerzeugnis als solches kein Vitamin $B_{12}$ enthält. Dabei nimmt die zusätzlich erzeugte Menge an Vitamin $B_{12}$ mit der Massenkonzentration des bei der Behandlung anwesenden Hefeerzeugnisses zumindest über einen bestimmten Konzentrationsbereich in etwa linear zu.

Jenseits einer Massenkonzentration des Hefeerzeugnisses von etwa 70 g/L, insbesondere jenseits von 50 g/L, (bezogen auf das erste Nährmedium) tritt jedoch eine starke Sedimentbildung im Reaktionsgefäß auf, und es kann zu Qualitätseinbußen bei dem erzeugten Nahrungsmittel (oder einer Vorstufe desselben) hinsichtlich des Geruchs und Geschmacks kommen. Dies ist vermutlich auf eine Überfrachtung mit Zellmaterial und deren Abbauprodukten zurückzuführen.

Wenn das Hefeerzeugnis eine frische oder getrocknete Hefe ist, kann es bei hohen Zugaben, vorzugsweise bei Zugaben jenseits der 20 g/L, insbesondere jenseits von 50 g/L, zu unangenehmer Geruchsentwicklung kommen. Dies ist vermutlich auf die verstärkte Freisetzung von Fettsäuren und/oder deren Abbauprodukten zurückzuführen.

Zudem wird die technische Umsetzung des beanspruchten Verfahrens beim Einsatz großer Mengen an Hefeerzeugnissen, vorzugsweise bei Massenkonzentrationen jenseits der 20 g/L, insbesondere jenseits der Konzentration von 50 g/L, zunehmend unwirtschaftlich.

Weiter kann, wenn der Schritt (b) in Anwesenheit eines Hefeerzeugnisses erfolgen soll, das Hefeerzeugnis vor der Durchführung des Schrittes (b) erwärmt werden bzw. worden sein, beispielsweise auf 80 °C oder mehr für 10 min oder länger. Die Erfinder haben herausgefunden, dass durch ein ausreichendes Erwärmen des Hefeerzeugnisses die Vitamin $B_{12}$-Konzentration im Medium nach der Behandlung gemäß Schritt (b) des erfindungsgemäßen Verfahrens erzielt werden kann gegenüber dem Fall, bei dem ein Erwärmen des Hefeerzeugnisses unterbleibt. Es wird vermutet, dass durch den Einfluss der Wärme die Zellwände der Hefezellen aufgeschlossen werden und/oder eine Eiweißdenaturierung im Hefeerzeugnis stattfindet, welche sich positiv auf die Vitamin $B_{12}$-Ausbeute auswirken könnte. Besonders vorteilhaft ist, wenn vor der Durchführung des Schrittes (b) das Hefeerzeugnis zusammen mit dem ersten Nährmedium, beispielsweise in Gestalt eines Gemischs, wie vorstehend beschrieben erwärmt wird. Hierdurch wird einerseits eine Sterilisierung des ersten Nährmediums, andererseits der vorstehend genannte Effekt der Steigerung der Vitamin $B_{12}$-Konzentration nach der Behandlung in einem Schritt bewirkt.

Es ist besonders vorteilhaft, wenn die Gewinnung des Hefeerzeugnisses einen Schritt umfasst, bei dem eine Hefe bei einer Temperatur zwischen 40 und 80 °C, vorzugsweise zwischen 50 und 70 °C, insbesondere zwischen 55 und 65 °C, inkubiert wird. Während des Inkubierens sollte ein Erwärmen der Hefe auf 80 °C oder mehr, vorzugsweise auf 70 °C oder mehr, insbesondere auf 65 °C oder mehr, (je nach Inkubationstemperatur) vorzugsweise für 5 min oder länger, insbesondere für 10 min oder länger, unterbleiben. Wenn ein Erwärmen der Hefe auf oder über die vorstehend angegebenen Temperaturen während des Inkubierens unterbleibt, so haben die Erfinder herausgefunden, kann eine höhere Vitamin $B_{12}$-Konzentration im Medium nach der Behandlung gemäß Schritt (b) des erfindungsgemäßen Verfahrens erzielt werden gegenüber dem Fall, bei dem ein Erwärmen des Hefe während des Inkubierens über die vorstehend angegebenen Temperaturen stattfindet. Es wird vermutet, dass zwar durch einen begrenzten Wärmeeinfluss (bis ca. 60 °C) die Zellwände der Hefezellen aufgeschlossen werden und/oder eine Eiweißdenaturierung in der Hefe bzw. im im Hefeerzeugnis stattfindet, welche sich positiv auf die Vitamin $B_{12}$-Ausbeute auswirken könnte. Wenn jedoch die thermische Belastung zu groß ist, wurde eine verringerte Vitamin $B_{12}$-Konzentration im Medium nach der Behandlung gemäß Schritt (b) gemessen. Ferner haben die Erfinder festgestellt, dass ein Erwärmen der Hefe am Ende des Inkubierens oder danach, beispielsweise zum Zwecke der Pasteurisierung, unschädlich für die Vitamin $B_{12}$-Ausbeute ist.

Weiter kann das erste Nährmedium frei oder im Wesentlichen frei von Hopfenbitterstoffen sein; und/oder das Hefeerzeugnis frei oder im Wesentlichen frei von Hopfenbitterstoffen sein.

Das erste Nährmedium und/oder das Hefeerzeugnis können dem Brauprozess entnommen werden. Insbesondere kann das erste Nährmedium eine Ausschlagwürze oder Anstellwürze sein. Ferner kann das Hefeerzeugnis eine Reinzuchthefe, beispielsweise angezogen auf gehopfter Würze, oder eine Erntehefe, beispielsweise aus der ersten, zweiten oder einer höheren Führung, sein. Für diese Fälle haben die Erfinder herausgefunden, dass im ersten Nährmedium und/oder im Hefeerzeugnis offenbar Hemmstoffe enthalten sein können, die für die Erzeugung von Vitamin $B_{12}$ mittels den vorgeschlagenen Milchsäurebakterienarten abträglich sein können. Wahrscheinlich handelt es hierbei um die Hopfenbitterstoffe.

Nach Erkenntnis der Erfinder können, beispielsweise durch ein- oder mehrmaliges Waschen des Hefeerzeugnisses mit Wasser, insbesondere Trinkwasser oder Brauwasser, die Hopfenbitterstoffe vollständig oder im Wesentlichen vollständig aus dem Hefeerzeugnis entfernt werden. Der Einsatz eines derartigen, von Hopfenbitterstoffen befreiten Hefeerzeugnisses im erfindungsgemäßen Verfahren führt zu einer Steigerung der Vitamin $B_{12}$-Erzeugung.

In ähnlicher Weise konnte eine wenigstens teilweise Hemmung der Vitamin $B_{12}$-Erzeugung im erfindungsgemäßen Verfahren festgestellt werden, wenn das erste Nährmedium nicht frei oder nicht im Wesentlichen frei von Hopfenbitterstoffen war. Vorteilhaft ist daher der Einsatz einer Maische oder ungehopften Würze, beispielsweise einer Vorderwürze oder ungehopften Ausschlag- oder Anstellwürze, als erstes Nährmedium, welche frei von Hopfenbitterstoffen sind.

Ferner kann das Hefeerzeugnis aus einer obergärigen oder untergärigen Hefe der Gattung *Saccharomyces,* insbesondere der Art *Saccharomyces cerevisiae* oder der Art *Saccharomyces carlsbergensis,* gewonnen werden. Dabei ist die Hefe vorzugsweise eine Reinzuchthefe oder eine Erntehefe aus dem Bierbereitungsverfahren. Ferner werden die Hopfenbitterstoffe aus der Hefe oder dem Hefeerzeugnis vollständig oder im Wesentlichen vollständig entfernt, vorzugsweise durch ein- oder mehrmaliges Waschen der Hefe oder des Hefeerzeugnisses mit Wasser, insbesondere mit Trinkwasser oder Brauwasser.

Indem das Hefeerzeugnis aus einer brauereiüblichen, obergärigen oder untergärigen Hefe gewonnen wird, insbesondere aus einer Erntehefe, steht im Falle der Durchführung des erfindungsgemäßen Verfahrens in einer Brauerei eine kostengünstige und mengenmäßig praktisch unbegrenzte Quelle für den Rohstoff des Hefeerzeugnisses zur Verfügung.

Darüber hinaus haben Untersuchungen gezeigt, dass ein unter Verwendung der genannten Hefen gewonnenes Hefeerzeugnis sich besonders positiv auf die Vitamin $B_{12}$-Erzeugung gemäß dem erfindungsgemäßen Verfahren auswirkt.

Zudem kann das Verfahren ferner die Schritte aufweisen:

(c) Bereitstellen einer Maische oder einer Würze als ein zweites Nährmedium; und

(d) Mischen des in Schritt (b) erhaltenen Mediums mit dem zweiten Nährmedium.

Durch das Mischen des in Schritt (b) erhaltenen Mediums mit einer Maische oder einer Würze als einem zweiten Nährmedium, kann durch einen einfachen, weiteren Verfahrensschritt eine biologisch gesäuerte Maische oder Würze erzeugt werden. Die technologischen Vorteile der biologischen Maische- oder Würzesäuerung sind dem Fachmann bekannt. Über die herkömmlichen Vorteile hinaus erlaubt das erfindungsgemäße Verfahren zudem die Gewinnung einer Maische oder Würze mit einem erhöhten Gehalt an bioverfügbarem Vitamin $B_{12}$.

Durch die Verwendung einer Maische oder Würze als zweitem Nährmedium wird vorteilhaft erzielt, dass das erzeugte Vitamin $B_{12}$ quantitativ im Wesentlichen erhalten bleibt und nicht von den Milchsäurebakterien wieder aufgenommen oder verstoffwechselt wird. Darüber hinaus bleibt das Vitamin $B_{12}$ im Wesentlichen vollständig oder wenigstens zu einem hohen Anteil bioverfügbar. Damit läßt sich eine Maische oder Würze erzeugen, die einen gegenüber herkömmlichen Maischen oder Würzen erheblich erhöhten Gehalt an Vitamin $B_{12}$ aufweist, ohne übersäuert zu sein.

So kann eine erfindungsgemäß erzeugte Maische einen pH-Wert im Bereich von 4,5 bis 5,7, vorzugsweise 4,9 bis 5,3, aufweisen. Eine erfindungsgemäß erzeugte Würze kann einen pH-Wert im Bereich von 4,2 bis 5,7, vorzugsweise 4,6 bis 5,0, aufweisen.

Die Einstellung des pH-Werts der Maische oder Würze auf die angegebenen Werte führt zu technologischen Vorteilen, wie beispielsweise verbesserte Geschmacksstabilität, Schaumstabilität und hellere Bierfarbe.

Das Verfahren kann ferner die Schritte aufweisen:

(e) vorzugsweise Läutern des in Schritt (b) oder (d) erhaltenen Mediums;

(f) Kochen oder Heißhalten und vorzugsweise Hopfung des in Schritt (d) oder (e) erhaltenen Mediums;

(g) wenigstens teilweises Entfernen von Trub aus dem in Schritt (f) erhaltenen Medium; und

(h) vorzugsweise Einstellen der Temperatur des in Schritt (g) erhaltenen Mediums auf eine Anstelltemperatur.

Durch die Anwendung der Schritte (e) bis (h) lässt sich die erfindungsgemäße, biologisch gesäuerte Maische oder Würze zu einer vergärbaren Ausschlag- und Anstellwürze weiterverarbeiten, welche ebenfalls einen erhöhten Gehalt an bioverfügbarem Vitamin $B_{12}$ aufweist.

So kann das Verfahren ferner wenigstens einen der Schritte aufweisen:

(i) Verarbeiten des in einem der Schritte (b), (d), (f), (g) oder (h) erhaltenen Mediums zu einem Getränk, vorzugsweise Behandeln dieses Mediums mit einer Hefe der Gattung *Saccharomyces,* insbesondere mit der Art *Saccharomyces cerevisiae* oder der Art *Saccharomyces carlsbergensis;* und/oder

(k) Mischen des in einem der Schritte (b), (d), (f), (g) oder (h) erhaltenen Mediums mit einem Getränk, vorzugsweise mit einem Bier.

Die in den verschiedenen Verfahrensschritten gewonnenen Vorstufen lassen sich erfindungsgemäß auf herkömmliche Weise, beispielsweise durch alkoholische und nicht-alkoholische Gärung, zu einem Getränk weiterverarbeiten, welches ebenfalls einen erhöhten Gehalt an bioverfügbarem Vitamin $B_{12}$ aufweist. Dieses Getränk kann insbesondere sein: ein alkoholfreies Getränks, ein Bier, insbesondere ein alkoholfreies Bier, ein Weizenbier, insbesondere ein alkoholfreies Weizenbier, ein bierhaltiges Getränk, insbesondere ein Biermischgetränk, ein mit einer Hefe fermentiertes oder nicht mit Hefe fermentiertes Getränk. Das erfindungsgemäße Getränk kann auch einfach durch Mischen der in den verschiedenen Verfahrensschritten gewonnenen Vorstufen mit einem Getränk, vorzugsweise mit einem Bier, gewonnen werden. Das erfindungsgemäße Getränk weist einen erhöhten Gehalt an bioverfügbarem Vitamin $B_{12}$ auf.

Trotz des erfindungsgemäß weiter gesteigerten Gehalts an Vitamin $B_{12}$ im fertigen Getränk findet nach Erkenntnis der Erfinder eine Beeinträchtigung der Bioverfügbarkeit bzw. einer Abreicherung in den weiteren Verfahrensschritten bis zum fertigen Getränk nicht oder im Wesentlichen nicht statt. Damit gelten dieselben Vorteile des erfindungsgemäß behandelten ersten Nährmediums, insbesondere des erzeugten Sauergutes, analog auch für das erfindungsgemäß hergestellte Getränk, insbesondere für ein Bier und ein alkoholfreies Bier.

Das Verfahren kann ferner den Schritt aufweisen:

(1) Verarbeiten des in einem der Schritte (b), (d), (f), (g) oder (h) erhaltenen Mediums oder des in Schritt (i) oder (k) erhaltenen Getränks zu einem nicht fluiden Nahrungsmittel;

wobei das in einem der Schritte (b), (d), (f), (g) oder (h) erhaltene Medium oder das in Schritt (i) oder (k) erhaltene Getränk mit einer Vorstufe des nicht fluiden Nahrungsmittels gemischt wird.

Die in den verschiedenen Verfahrensschritten gewonnenen Vorstufen (behandelte Medien) oder das Getränk lassen sich erfindungsgemäß auf herkömmliche Weise, beispielsweise durch Einengen und/oder Mischen mit anderen Bestandteilen, zu einem nicht fluiden Nahrungsmittel weiterverarbeiten, welches ebenfalls einen erhöhten Gehalt an bioverfügbarem Vitamin $B_{12}$ aufweist. Dieses Nahrungsmittel kann insbesondere sein: ein getreidehaltiges Nahrungsmittel, insbesondere ein getreidehaltiger Riegel, Frühstückscerealien, ein Malzextraktprodukt, eine Backware, ein Milchprodukt, insbesondere ein Joghurt.

Damit gelten die Vorteile des erfindungsgemäß hergestellten Getränks für das vorstehend erläuterte, erfindungsgemäße nicht fluide Nahrungsmittel analog.

Der Massenanteil an Wasser im Medium, welches in einem der Schritte (b), (d), (f), (g) oder (h) erhalten wurde, im in Schritt (i) oder (k) erhaltenen Getränk oder im in Schritt (1) erhaltenen, nicht fluiden Nahrungsmittel kann auf weniger als 35 %, vorzugsweise weniger als 30 %, vorzugsweise weniger als 25 %, vorzugsweise auf weniger als 20 %, insbesondere auf weniger als 15 %, eingestellt werden.

Höhere als die vorstehend angegebenen Wasseranteile erlauben nicht die Sicherstellung der mikrobiologischen Stabilität des mit dem erfindungsgemäßen Verfahren erzeugten Nahrungsmittels oder einer Vorstufe desselben. Wird der Wassergehalt durch Wasserentzug eingestellt, werden aufgrund der Volumenverringerung die Transport- und Lagerkosten für das konzentrierte Medium verringert.

Darüber hinaus kann das konzentrierte Medium überall und unabhängig vom Herstellungsort durch Rückverdünnung auf die Ursprungskonzentration oder eine gewünschte Konzentration konfektioniert werden.

Ferner weist das konzentrierte Medium eine gegenüber dem Ausgangsstoff erhöhte Viskosität auf, die bei der Verwendung des konzentrierten Mediums zur Herstellung von Nahrungsmitteln vorteilhaft ist. So kann das konzentrierte Medium beispielsweise als Bindemittel für körnige oder pulverige Nahrungsmittelbestandteile dienen.

Ferner ist es vorteilhaft, wenn der Massenanteil an Wasser im erfindungsgemäß erzeugten Medium oder Getränk auf mehr als 0 %, insbesondere mehr als 5 %, eingestellt wird.

Durch das Einstellen eines definierten Restwassergehaltes wird die Staubbildung bei der Verarbeitung oder Handhabung des Nahrungsmittels oder der Vorstufen desselben vermieden.

Die erfindungsgemäße Aufgabe wird ferner durch den Gegenstand des Erzeugnisanspruchs 11 gelöst. Darin wird ein Nahrungsmittel oder eine Vorstufe desselben beansprucht, das mit einem Verfahren nach einem der Ansprüche 1 bis 10 hergestellt wird.

Dabei gelten die bei der Beschreibung des erfindungsgemäßen Herstellungsverfahrens genannten Vorteile für ein mittels dieses Verfahrens erzeugtes Nahrungsmittel oder eine Vorstufe desselben, insbesondere ein Sauergut, eine Maische, eine Würze, ein Getränk oder ein nicht fluides Nahrungsmittel, analog.

Die erfindungsgemäße Aufgabe wird ferner durch den Gegenstand des Verwendungsanspruchs 12 gelöst.

Darin wird die Verwendung von Milchsäurebakterien der Art *Lactobacillus rossiae* (DSM 15814T) oder von Milchsäurebakterien wenigstens umfassend zwei Arten, darunter *Lactobacillus rossiae* (DSM 15814T), vorzugsweise eines Gemisches aus Milchsäurebakterien der Arten *Lactobacillus rossiae* (DSM 15814T) und *Lactobacillus coryniformis,* insbesondere der Unterart *Lactobacillus coryniformis* subsp. *coryniformis* (DSMZ Nr. 20007), zur Herstellung bzw. in einem Verfahren zur Herstellung eines Nahrungsmittels oder einer Vorstufe desselben, vorzugsweise in einem Verfahren nach einem der Ansprüche 1 bis 10, vorzugsweise zum Zwecke der Steigerung der Erzeugung von Vitamin $B_{12}$, insbesondere der Steigerung der Erzeugung von bioverfügbarem Vitamin $B_{12}$, beansprucht. Dabei weist das Verfahren wenigstens die Schritte auf:

(a) Bereitstellen einer Maische oder einer Würze oder eines Glattwassers als ein erstes Nährmedium; und

(b) Behandeln des ersten Nährmediums mit Milchsäurebakterien der Art *Lactobacillus rossiae* (DSM 15814T) oder mit Milchsäurebakterien wenigstens umfassend zwei Arten, darunter *Lactobacillus rossiae* (DSM 15814T), vorzugsweise mit einem Gemisch enthaltend Milchsäurebakterien der Arten *Lactobacillus rossiae* (DSM 15814T) und *Lactobacillus coryniformis,* insbesondere der Unterart *Lactobacillus coryniformis* subsp. *coryniformis* (DSMZ Nr. 20007).

Dabei findet die Behandlung des Schrittes (b) vorzugsweise in Anwesenheit des Hefeerzeugnisses statt, wobei das Hefeerzeugnis ein vorzugsweise Extrakt, ein Autolysat und/oder eine Hefe in frischer oder getrockneter Gestalt enthält. Ferner enthält das Hefeerzeugnis vorzugsweise ein Extrakt, ein Autolysat und/oder eine Hefe in frischer oder getrockneter Gestalt. Das Hefeerzeugnis kann auch aus einem Extrakt, einem Autolysat und/oder einer Hefe in frischer oder getrockneter Gestalt bestehen.

Vorteilhafte Ausführungsformen der erfindungsgemäßen Verwendung sind Gegenstand der abhängigen Ansprüche. Einzelne oder mehrere der vorstehend genannten Merkmale des erfindungsgemäßen Verfahrens lassen sich auch mit der erfindungsgemäßen Verwendung kombinieren. Dabei gelten die Vorteile der entsprechenden Merkmale des erfindungsgemäßen Verfahrens analog.

So kann in einer bevorzugten Ausführungsform der erfindungsgemäßen Verwendung das Hefeerzeugnis in einer Massenkonzentration im Bereich von $\geq 0,2$ bis $\leq 70$ g/L, vorzugsweise von $\geq 8$ bis $\leq 50$ g/L, bezogen auf das erste Nährmedium vorliegen.

Ferner kann das erste Nährmedium frei oder im Wesentlichen frei von Hopfenbitterstoffen sein. Zusätzlich oder alternativ kann das Hefeerzeugnis frei oder im Wesentlichen frei von Hopfenbitterstoffen sein.

Alternativen und weitere Offenbarung der Erfindung

Die Erfindung ist nicht auf die Verwendung von *Lactobacillus rossiae* (DSM 15814$^T$ bzw. 15814) gemäß Anspruch 1 und insbesondere nicht auf die Kombination von *Lactobacillus rossiae* (DSM 15814$^T$) und *Lactobacillus coryniformis* gemäß Anspruch 2 beschränkt. Die vorstehend beschriebenen Herstellungsverfahren, Verwendungen sowie Erzeugnisse lassen sich alternativ auch mit einer Kombination von *Lactobacillus rossiae* (DSM 15814$^T$) mit Milchsäurebakterien der Arten *Lactobacillus backii* (DSM 18080), *Lactobacillus plantarum* (DSM 2648, DSM 2601, DSM 20174, DSM 13273) oder *Lactobacillus fermentum* (DSM 20052), also einem Gemisch aus Milchsäurebakterien von *Lactobacillus rossiae* (DSM 15814$^T$) und von wenigstens einer der anderen genannten Arten oder derer Unterarten durchführen bzw. herstellen. Hierdurch werden die vorstehend beschriebenen Vorteile und Eigenschaften in analoger Weise verwirklicht.

Vorteilhaft kann eine Vorstufe des Hefeerzeugnisses, insbesondere eine obergärige oder untergärige Reinzuchthefe oder Erntehefe aus der Brauerei, mit Wasser oder einem anderen geeigneten Stoff ein- oder mehrfach gewaschen werden. Dies geschieht beispielsweise durch Suspendieren der Hefezellen im Wasser und Abzentrifugieren. Nach Verwerfen des Überstandes kann die derart gewaschene Hefemasse erneut in Wasser suspendiert und anschließend wieder abzentrifugiert werden. Die Schritte des Resuspendierens und des Abzentrifugierens können so oft wiederholt werden, bis ein gewünschter Reinheitsgrad erreicht ist, insbesondere bis die Hefe frei oder im Wesentlichen frei von Hopfenbitterstoffen ist.

Vorteilhaft wird als erstes Nährmedium eine Vorderwürze aus der Brauerei gewählt, wobei die Vorderwürze in Schritt (a) einen Extraktgehalt im Bereich von 7 bis 28 %, vorzugsweise 10 bis 22 %, vorzugsweise 12 bis 19 %, insbesondere 14 bis 16 %, aufweist.

Vorderwürze als erstes Nährmedium zeichnet sich durch einen hohen Gehalt an für die im erfindungsgemäßen Verfahren verwendeten Milchsäurebakterien erforderlichen Nährstoffen aus. Darüber hinaus ist Vorderwürze gut verfügbar und mit den Anlagen einer herkömmlichen Brauerei einfach zu erzeugen.

Zudem kann eine große Konzentrationsbreite hinsichtlich des Extraktgehaltes der Vorderwürze eingesetzt werden, wodurch das Verfahren in der Anwendung flexibel ist.

Das erfindungsgemäße Verfahren ist auch bei hochkonzentrierten Nährmedien, welche beispielsweise beim High-Gravity-Verfahren gewonnen werden, unter Erzielung der damit verbundenen Vorteile, insbesondere Volumen- und Kostenersparnis, anwendbar.

In einer vorteilhaften Ausführungsform wird das erste Nährmediums vor dem Behandeln mit den Milchsäurebakterien (Schritt (b)) mit Wasser, insbesondere Brauwasser, derart verdünnt, dass der Extraktgehalt der resultierenden Verdünnung im Bereich von 5 bis 16 %, vorzugsweise 10 bis 15 %, insbesondere 12 bis 14 %, beträgt.

Durch eine Verdünnung des ersten Nährmediums kann die für die im erfindungsgemäßen Verfahren verwendeten eingesetzten Mikroorganismen optimale Nährstoffkonzentration eingestellt werden.

Zudem kann der Verbrauch an dem zu erzeugenden ersten Nährmedium verringert werden.

Das erste Nährmedium kann mit den Milchsäurebakterien in einer Menge angeimpft werden, so dass die optische Dichte (OD) des ersten Nährmediums unmittelbar nach dem Animpfen im Bereich von etwa 0,1 bis 1,0 OD, vorzugsweise 0,2 bis 0,8, vorzugsweise 0,3 bis 0,7, vorzugsweise 0,4 bis 0,5 OD, liegt, insbesondere etwa 0,45 OD beträgt, wobei der Messwert der optischen Dichte bei einer Wellenlänge von 620 nm gemessen wird und um den Einfluss des ersten Nährmediums bereinigt ist.

Das Animpfen des ersten Nährmediums mit den erfindungsgemäß verwendeten Mikroorganismen derart, dass die vorstehend erwähnte optische Dichte (OD) oder eine entsprechende Zelldichte zu Beginn der Behandlung eingestellt wird, führt zu einer schnellen Umsetzung und damit zu einer vorteilhaften kurzen Behandlungszeit.

Darüber hinaus führt eine optimale Animpfkonzentration der Milchsäurebakterien zu einem optimalen Aromaprofil mit minimalen Konzentrationen an Fehlaromen oder deren vollständiger Abwesenheit.

Eine optische Dichte des ersten Nährmediums unmittelbar nach dem Animpfen und gegebenenfalls Homogenisieren von weniger als 0,4, vorzugsweise 0,3, vorzugsweise 0,2 und insbesondere 0,1 erfordert nachteilig eine zu lange Zeitdauer bis eine angestrebte, hohe Stoffumsatzrate der Milchsäurebakterien erreicht ist.

Dagegen bewirkt eine optische Dichte des ersten Nährmediums unmittelbar nach dem Animpfen und gegebenenfalls Homogenisieren von mehr als 0,5, vorzugsweise 0,7, vorzugsweise 0,8, insbesondere 1,0 nicht optimale Wachstumsbedingungen für die Milchsäurebakterien, beispielsweise wegen Feedback-Hemmung.

Die Milchsäurebakterien können sich zu Beginn der Behandlung des ersten Nährmediums, insbesondere bei deren Zugabe zum ersten Nährmedium, gemäß Schritt (b) in der log-Phase (logarithmische Phase) bzw. Wachstumsphase befinden.

Ein Animpfen mit Mikroorganismen, welche sich in der log-Phase bzw. Wachstumsphase befinden, führt aufgrund der hohen Aktivität der eingesetzten Mikroorganismen vorteilhaft zu einer schnellen Umsetzung des ersten Nährmediums zum Sauergut.

Die Dauer der Behandlung gemäß Schritt (b) kann zwischen etwa 5 und 80 Stunden, vorzugsweise 15 bis 60, insbesondere 20 bis 50 Stunden, insbesondere 20 bis 30 Stunden, betragen.

Der erfindungsgemäß vorgesehene Behandlungszeitraum kann vorteilhaft auf die vorstehend angegebenen, kurzen Zeitdauern beschränkt werden. Hierdurch kann eine kurzfristige Bereitstellung eines behandelten ersten Nährmediums, insbesondere eines Sauerguts, realisiert werden. Insbesondere ist bei einer Behandlungsdauer von weniger als 5 Stunden die Ausbeute an Vitamin $B_{12}$ und/oder Laktat zu gering. Dagegen wird bei einer Behandlungsdauer von mehr als 60 Stunden, insbesondere mehr als 80 Stunden, keine weitere Steigerung der Erzeugung von bioverfügbarem Vitamin $B_{12}$ erzielt. Ferner besteht die Gefahr der Übersäuerung.

Die Behandlung gemäß Schritt (b) kann bei einer Temperatur des Nährmediums im Bereich von etwa 15 bis 48 °C, vorzugweise 25 bis 42 °C, insbesondere 30 bis 40 °C, insbesondere 35 bis 39 °C, insbesondere 36 bis 38 °C, durchgeführt werden.

Die Behandlung gemäß Schritt (b) kann vorteilhaft in einem breiten Temperaturbereich erfolgen. Eine Auswahl der Temperatur zwischen etwa 30 und 40 °C schafft optimale Wachstumsbedingungen für die verwendeten Mikroorganismen, wodurch sich die erforderliche Behandlungszeit verkürzt und eine optimale Qualität des resultierenden Erzeugnisses erhalten wird.

Das Verfahren kann ferner einen Schritt aufweisen, in dem das gemäß Schritt (b) behandelte erste Nährmedium sterilisiert wird.

Durch einen abschließenden Sterilisationsschritt kann ausgeschlossen werden, dass die erfindungsgemäß eingesetzten Milchsäurebakterien bei der weiteren Verwendung des Erzeugnisses, beispielsweise im Rahmen einer Nahrungsmittel- oder Getränkeherstellung, insbesondere bei der Bierherstellung, einen unerwünschten Einfluss auf das jeweilige Erzeugnis haben können.

So wird insbesondere bei einem Einsatz von Hefe in einem späteren Verfahrensschritt deren Stoffwechselaktivitäten nicht negativ beeinflusst.

Die Sterilisation kann damit mit allen dem Fachmann bekannten Mittel erfolgen. Bevorzugt ist dabei die Zugabe des Sauerguts zur kochenden oder heißen Würze.

Das in einem der Schritte (b), (d), (f), (g) oder (h) erhaltene Medium, das in Schritt (i) oder (k) erhaltene Getränk oder das in Schritt (1) erhaltene, nicht fluide Nahrungsmittel kann einen Massenanteil von Milchsäure im Bereich von etwa 0,1 bis 1,0 %, vorzugweise 0,2 bis 0,6 %, vorzugsweise etwa 0,3 bis 0,5 %, insbesondere etwa 0,35 bis 0,45 %, aufweisen.

Durch das Vorhandensein von Milchsäure in den angegebenen Massenanteilen lässt sich das erfindungsgemäß erzeugte Medium, insbesondere das Sauergut, vorteilhaft zur Einstellung eines bestimmten pH-Wertes, beispielsweise von Maische oder Würze, verwenden. Hierdurch lässt sich auf natürliche Weise und entsprechend dem Reinheitsgebot eine pH-Wert-Einstellung von Nahrungsmitteln oder entsprechenden Vorstufen derselben, insbesondere von Maische oder Würze, bewerkstelligen.

Das zweite Nährmedium kann bei Durchführung des Schritts (d) eine Temperatur von wenigstens 50 °C, vorzugsweise wenigstens 60 °C, vorzugsweise wenigstens 70 °C, vorzugsweise wenigstens 80 °C, insbesondere wenigstens 90 °C, insbesondere wenigstens 95 °C, aufweisen.

Durch die Zugabe des in Schritt (b) erhaltenen Mediums zum zweiten Nährmedium bei einer hohen Temperatur wird eine wirksame Inaktivierung bzw. Sterilisation wenigstens eines Teils der Milchsäurebakterien in einem Schritt erzielt. Hierdurch entfällt ein gesonderter Sterilisationsschritt sowie der damit verbundene Kosten-, Zeit- und Energieaufwand.

Das in Schritt (b) erhaltene Medium kann in einem Volumenanteil von 2 bis 20 %, vorzugsweise von 5 bis 15 %, vorzugsweise 6 bis 12 %, vorzugsweise 7 bis 11 %, insbesondere 8 bis 10 %, bezogen auf das Volumen der resultierenden Mischung, zugegeben werden.

Durch Einstellung eines derartigen Volumenanteils des Sauerguts an der resultierenden Mischung kann ein für die Maische bzw. Würze optimaler pH-Wert, vorzugsweise ein pH-Wert der resultierenden Mischung im Bereich von 4,2 bis 5,5, vorzugsweise 4,6 bis 5,3, erzielt werden.

Das Behandeln des ersten Nährmediums mit Milchsäurebakterien gemäß Schritt (b) kann unter im Wesentlichen anaeroben Bedingungen, insbesondere unter anaeroben Bedingungen, stattfinden.

Möglicherweise hat die Wahl von anaeroben Bedingungen bzw. im Wesentlichen anaeroben Bedingungen während der Behandlung des Nährmediums mit den erfindungsgemäß eingesetzten Milchsäurebakterien eine positive Auswirkung auf Quantität und/oder Bioverfügbarkeit des erzeugten Vitamins $B_{12}$.

Bevorzugt werden anaeroben Bedingungen durch Luftabschluß und/oder Begasen mit $CO_2$ oder $N_2$ oder anderen bekannten Maßnahmen hergestellt. Erfindungsgemäß werden unter "anaeroben Bedingungen" ein Sauerstoffgehalt von höchstens 0,1 mg/L, vorzugsweise 0 mg/l, im Medium und/oder in der darüber stehenden Gasphase verstanden. Erfindungsgemäß werden unter "im Wesentlichen anaeroben Bedingungen" ein Sauerstoffgehalt von höchstens 0,5 mg/L, vorzugsweise höchstens 0,1 mg/l, im Medium und/oder in der darüber stehenden Gasphase verstanden und schließt die Bedingungen, die der Fachmann unter "mikroaerophil" versteht mit ein.

Das erfindungsgemäß erzeugte Nahrungsmittel, insbesondere das Getränk, kann konform mit dem deutschen Reinheitsgebot sein oder ausschließlich aus Zutaten hergestellt werden, welche nach dem deutschen Reinheitsgebot für die Herstellung von Bier zugelassen sind. So können die Rohstoffe des erfindungsgemäßen Nahrungsmittels bzw. Getränks auf die gemäß dem deutschen Reinheitsgebot zugelassenen Rohstoffe zum Bierbrauen, insbesondere Gerstenmalz, Weizenmalz, Hopfen, Hopfenprodukte, insbesondere Hopfenextrakte, und Brauwasser, und die erfindungsgemäß eingesetzten Mikroorganismen einschließlich Hefen der Gattung *Saccharomyces,* beschränkt werden. Damit wird es erfindungsgemäß erstmalig möglich, ein Nahrungsmittel, insbesondere ein Getränk, mit bioverfügbarem Vitamin $B_{12}$ in erhöhter Massenkonzentration bereitzustellen, das dem Reinheitsgebot entspricht.

In einer vorteilhaften Ausführungsform des erfindungsgemäßen Verfahrens kann in einem weiteren Schritt das bei der Behandlung gemäß Schritt (b) entstandene Lactat teilweise oder vollständig durch Mikrofiltration, Dialyse oder ein sonstiges, geeignetes Trennverfahren abgetrennt werden. Hierdurch wird vorteilhaft der saure Geschmackseindruck gemindert oder ganz vermieden, wodurch sich der Einsatzbereich des resultierenden Mediums weiter verbreitert.

Das erfindungsgemäß erzeugte Nahrungsmittel kann eine gel- oder pastenartige Konsistenz aufweisen. Eine gel- oder pastenartige Konsistenz begünstigt vorteilhaft eine schnellere bzw. bessere Resorption der darin enthaltenen Nährstoffe, insbesondere des Vitamins $B_{12}$, im menschlichen oder tierischen Körper. Ferner bewirkt eine gel- oder pastenartige Konsistenz eine verbesserte Verträglichkeit und eine leichte Verzehrbarkeit bzw. Handhabbarkeit, beispielsweise während des Konsums bei Sport- oder Freizeitaktivitäten.

Das erfindungsgemäß erzeugte Nahrungsmittel kann einen Massenanteil an Vitamin $B_{12}$ von wenigstens 0,15 $\mu$g pro Portion, vorzugsweise wenigstens 0,2 $\mu$g pro Portion, vorzugsweise wenigstens 0,3 $\mu$g pro Portion, vorzugsweise wenigstens 0,35 $\mu$g pro Portion, vorzugsweise wenigstens 0,4 $\mu$g pro Portion, vorzugsweise wenigstens 0,5 $\mu$g pro Portion, vorzugsweise wenigstens 0,6 $\mu$g pro Portion, vorzugsweise wenigstens 1,0 $\mu$g pro Portion, insbesondere wenigstens 1,5 $\mu$g pro Portion des Nahrungsmittels aufweist, wobei eine Portion des Nahrungsmittels eine Masse von 20 g aufweist.

Je höher der Massenanteil an Vitamin $B_{12}$ im erfindungsgemäßen Nahrungsmittel, desto besser ist die erzielbare Versorgung des menschlichen oder tierischen Körpers mit Vitamin $B_{12}$.

Das erfindungsgemäß erzeugte Nahrungsmittel kann Getreidebestandteile, vorzugsweise vermälztes und/oder unvermälztes Braugetreide, insbesondere Gerstenmalz und/oder Weizenmalz, enthalten.

Insbesondere bei der Verwendung von Maische oder Würze als erstem Nährmedium, hat die Anmelderin überraschend festgestellt, dass die erfindungsgemäß eingesetzten Milchsäurebakterien in einem derartigen Milieu Vitamin $B_{12}$ erzeugen und damit zu einer Erzeugung von Vitamin $B_{12}$ geeignet sind. Ferner wurde überraschend festgestellt, dass das mittels der erfindungsgemäß eingesetzten Milchsäurebakterien erzeugte Vitamin $B_{12}$ auch wenigstens zu einem hohen Anteil bioverfügbar in Sinne dieser Anmeldung ist. Auch bei der Anwendung des vorstehend definierten, alternativen Verfahrens kann also eine aktive Erzeugung von bioverfügbarem Vitamin $B_{12}$ erreicht werden, offensichtlich ohne dass ein "Umschalten" des Stoffwechsels der erfindungsgemäß vorgesehenen Milchsäurebakterien auf die Erzeugung von nicht-bioverfügbarem Vitamin $B_{12}$ stattfindet.

Die in Zusammenhang mit der in dieser Anmeldung beschriebenen Erfindung genannten Merkmale stellen, falls nicht anders erwähnt oder ersichtlich, fakultative Merkmale vorteilhafter Ausführungsformen dar, welche sich beliebig mit den hierin beschriebenen Gegenständen und untereinander kombinieren lassen, soweit der Fachmann hierin kein offensichtliches Hindernis sieht. So lassen sich insbesondere alle für die in dieser Beschreibung angegebenen Merkmale der Verfahren auch mit den in dieser Anmeldung beschriebenen Erzeugnissen verknüpfen, und umgekehrt. Insbesondere sind alle in Zusammenhang mit einem erfindungsgemäßen Erzeugnis genannten Merkmale auf alle weiteren, in dieser Anmeldung beschriebenen Erzeugnissen übertragbar und damit verknüpfbar. Analoges gilt für alle in dieser Anmeldung beschriebenen Verfahren und deren Merkmale. Analoges gilt für die mittels den beschriebenen Merkmalen erzielten Wirkungen und Vorteile.

Ausführungsbeispiele

1. Befreien der Hefe von Hemmstoffen, insbesondere von Hopfenbitterstoffen, durch Waschen

Eine obergärige oder untergärige Reinzuchthefe oder Erntehefe wird mit Brauwasser im Verhältnis 1:9 (50 g Betriebshefe + 400 ml Wasser) suspendiert. Die entstandene Suspension wird 5 min bei 1000 G zentrifugiert. Anschließend wird der Überstand verworfen und das Hefesediment in 150 ml Brauwasser resuspendiert. Die beiden letzten Schritte können zwei- bis dreimal wiederholt werden.

Die Hefe weist nach dem Waschen einen reinen, frischen, fruchtigen Geruch auf. Es fehlt die ursprünglich vorhandene Bitternote. Im mikroskopischen Präparat erscheinen die Hefezellen intakt. Lediglich im Falle der untergärigen Hefe treten vereinzelt geschädigte Zellen auf. Die gewaschenen Hefezellen weisen neben einem großen, runden Zellkern ein homogenes Plasma auf. Es werden keine Zellwandaufbrüche beobachtet. Nach Vitalfärbung sind die Zellen weiterhin farblos (= lebend).

2. <u>Herstellen eines Hefeautolysats</u>

Der Vorgang der Hefeautolyse wird vorzugsweise durch das Aufbrechen der Zellen eingeleitet. Hierfür werden die Hefen mechanisch, thermisch oder chemisch behandelt. Die Autolyse läuft dann während einer mehrstündigen bis mehrtägigen Inkubation der Hefe bei 40 bis 55 °C und bei geeignetem pH-Wert, vorzugsweise bei einem pH-Wert im Bereich von 5 bis 7, ab.

Eine obergärige oder untergärige Reinzuchthefe oder Erntehefe wird in Ausschlagwürze vermehrt. Die Hefe weist einen Trockensubstanzanteil von etwa 16 bis 17 % auf. Sie wird frisch geerntet und nach dem vorstehend beschriebenen Verfahren gewaschen.

Optional können die Hefezellen zum Aufbrechen durch eine oder mehrere der nachfolgenden Maßnahmen vorbehandelt werden:

a) Nasszellaufschluss mittels Hochdruckhomogenisator;
b) Ultraschallbehandlung (mit und ohne Glaskugeln);
c) Vortexen (mit und ohne Glaskugeln); und
d) Zugabe von Propionsäure.

Details zur einzelnen Vorbehandlungsverfahren werden unten erläutert.

Die eigentliche Autolyse der Hefezellen findet dann durch Inkubieren der ggf. vorbehandelten Hefenzellen für 24 Stunden bei ca. 53 °C im Brutschrank (Regelamplitude: 50 bis 55 °C) unter permanenten Rühren des Ansatzes statt. Im Ergebnis entsteht ein flüssiges Autolysat.

3. <u>Herstellen eines Hefeextrakts (Variante aus Autolysat)</u>

Das wie vorstehend beschrieben hergestellte, flüssige Autolysat wird durch Wasserentzug aufkonzentriert. Bei Bedarf kann es filtriert und von geschmacklich störenden Stoffen befreit werden.

Die Hauptbestandteile des so gewonnenen Hefeextrakts sind Peptide und Aminosäuren als Ergebnis des Eiweißabbaus sowie Purine und Pyrimidine, die bei der enzymatischen Spaltung der Nukleinsäuren entstehen.

4. <u>Details zur Vorbehandlung</u>

a) <u>Nasszellaufschluss mittels Hochdruckhomogenisator</u>

Der mechanische Aufschluss der Hefezellen wurde mit dem Hochdruckhomogenisator PANDA Plus 2000 der Firma GEA Niro Soavi Deutschland durchgeführt. Der Nasszellaufschluss mittels Hochdruckhomogenisator stellt das bevorzugte Aufschlußverfahren dar.

Als Folge einer speziellen Strömungsdynamik entsteht im verwendeten Homogenisator ein örtlicher statischer Unterdruck (500 bis 1.500 bar, vorzugsweise 800 bis 1.200 bar). Hierdurch kommt es zu einer Blasenbildung sowohl innerhalb der Hefezelle als auch an der Grenzfläche zwischen der Hefezellwand und dem umgebenden Medium (Kavitationseffekt). Wird der Unterdruck später an einem Entspannungsventil spontan wieder abgebaut, so führt das zum Implodieren der Bläschen. Dies zieht eine punktuelle Ruptur der Zellwände nach sich.

Zur Vorbereitung der Hefeproben wird die frisch gezogene Betriebshefe mit Brauwasser verdünnt (1:2, v/v), auf dem Magnetrührer entkohlensäuert und anschließend dreimal gemäß dem vorstehend beschriebenen Verfahren gewaschen.

Jede Hefeprobe durchläuft das beschriebene Aufschlussverfahren zweimal. Die lichtmikroskopische Betrachtung der derart behandelten Zellen ergibt, dass die Zellen nach Anwendung des Homogenisators keinen Protoplasten mehr enthalten und im Präparat nur die Zellhüllen verbleiben.

Die derart aufgeschlossene Hefe wird anschließend bei etwa 53 °C für etwa 24 Stunden im Brutschrank inkubiert und durch die noch intakten Enzyme autolysiert. Um die Autolyse zu stoppen, werden die Ansätze für etwa 30 min gekocht.

Alternativ können zum Zellaufschluß die nachfolgenden Verfahren eingesetzt werden:

b) <u>Ultraschall (mit und ohne Glaskugeln)</u>

Die Hefeprobe wird mit Brauwasser (10:90, v/v) verdünnt und anschließend mit Ultraschall für 10 Minuten beaufschlagt (Ultraschallbad: MERCK eurolab USR 46 H).

Zur Verstärkung der mechanischen Kräfte können der Hefeprobe während der Ultraschallbehandlung kleine Glasperlen (Fa. Prolabo/VWR, Durchmesser von 2,5 bis 3,5 mm) zugesetzt werden.

c) Vortexen mittels Reagenzglasschüttler (mit und ohne Glaskugeln)

Die Hefeprobe wird mit Brauwasser (10:90, v/v) verdünnt und anschließend eine Minute lang mittels Reagenzglasschüttler intensiv geschwenkt bzw. gerührt ("Vortexen"; Vortex Genie 2-Schüttler: Bender & Hobein AG, Stufe 8).

Zur Verstärkung der mechanischen Kräfte können der Hefeprobe kleine Glasperlen (Fa. Prolabo/VWR, Durchmesser von 2,5 bis 3,5 mm) während des Vortexens zugesetzt werden.

d) Zugabe von Propionsäure

Die Hefeprobe wird mit Propionsäure derart versetzt, dass der Anteil der Säure in der Mischung 5 vol.-% beträgt. Der Ansatz wird von Hand und dann mittels Vortexer geschwenkt. Anschließend wird der Ansatz für 15 Minuten über Kopf geschüttelt (TURBULA-Schüttler). Die Proben werden für 2 Stunden bei Raumtemperatur belassen und wiederholt geschwenkt.

5. Herstellen von getrockneter Hefe und Hefeflocken

Die Hefesuspension mit einem Trockensubstanzanteil von etwa 15 % wird gleichmäßig auf eine heiße Walze gesprüht (Walzentrockner der Fa. VITAM GmbH, Hameln). Beim Kontakt mit der Walzenoberfläche platzen die Hefezellen. Zellwand und Zellinhalt trocknen an der Walze an und werden nach spätestens 3 Sekunden als Flocken abgeschabt. 10 L Hefesuspension liefern etwa 1,5 kg Flocken. Anschließend werden die Hefeflocken im Mörser zerkleinert.

Alle vorstehend genannten und weitere, kommerziell verfügbare Hefeerzeugnisse wurden auf ihren Gehalt an Vitamin $B_{12}$ überprüft: Keines der Hefeerzeugnisse wies eine nachweisbare Mengen an Vitamin $B_{12}$ auf.

Herstellung des Hefeextraktes (Variante 1 aus Trockenhefe)

In einer 500-mL-Flasche werden 20 g einer Trockenhefe des Stammes W34/70 (Fa. Fermentis, Marcq en Baroeul/Frankreich) in 280 mL kurzzeiterhitzter Würze suspendiert. Anschließend wird der Ansatz 30 min stehen lassen und dann 30 min schwach gerührt (Stufe 1 Magnetrührplatte).

Der wie vorstehend beschriebene Ansatz wird bei ca. 57 °C für etwa 72 Stunden inkubiert (Wärmestress). Dabei ist die Flasche ein wenig geöffnet, damit entstehendes Gas entweichen kann.

Der so erhaltene Ansatz wird bei 4500 g für 5 bis 10 min zentrifugiert. Anschließend wird der Überstand in ein steriles Gefäß überführt. Bei noch zu trübem Überstand wird erneut zentrifugiert. Der auf diese Weise erhaltene Überstand (ca. 250 mL) ist der Hefeextrakt gemäß dieser Ausführungsform.

Herstellung des Hefeextraktes (Variante 2 aus Trockenhefe)

In einen Tank werden 33,3 kg einer Trockenhefe des Stammes W34/70 (Fa. Fermentis, Marcq en Baroeul/Frankreich) mit einem Trockensubstanzgehalt von 20 % in 150 L Vorderwürze suspendiert. Anschließend wird der Ansatz 30 min stehen lassen.

Die hierzu verwendete Vorderwürze ist eine ungehopfte Vorderwürze mit 16,5 ° Plato, welche aus einer Maische mit einer Schüttung von mindestens 50 % Weizenmalz gewonnen wurde. Die Vorderwürze wird bei 85 °C für etwa 10 min pasteurisiert und anschließend auf Raumtemperatur gekühlt.

Eine für die Suspendierung der Trockenhefe nicht verwendete Menge der wie vorstehend beschrieben hergestellten und pasteurisierten Vorderwürze (ca. 350 L) wird in einem Braun-Fermenter auf ca. 57 °C erwärmt. Dieser erwärmten Vorderwürze wird die wie vorstehend beschrieben hergestellte Hefesuspension zugegeben, woraus ein Gesamtvolumen von ca. 500 L des Gemischs resultiert. Der Fermenter weist ein Steigvolumen von ca. 50 % auf. Das Gemisch wird bei 57 °C für etwa 72 Stunden inkubiert bzw. fermentiert.

Anschließend wird der Fermenterinhalt bei 85 °C für etwa 10 min pasteurisiert und anschließend auf etwa 5 °C gekühlt. Der gekühlte Ansatz wird mittels Mikrofiltration oder Zentrifugation von toten Hefezellen und anderen Feststoffen befreit, woraus das verwendungsfertige Hefeextrakt entsteht. Das Hefeextrakt kann anschließend bei 5 °C in einem sterilen Tank bis zur Verwendung gelagert werden.

Anzucht der Laktobazillen (Variante 1): *Lactobacillus rossiae* (DSM 15814)

Es wurden 500 mL MRS-Medium (Fa. Merck, Darmstadt) angesetzt und bei 118 °C für 15 min autoklaviert. Ein Lyophilisat der Lactobazillen wurde im abgekühlten MRS-Medium suspendiert und bei 30 °C für 2 Tage inkubiert. Der so erhaltene Ansatz wurde im Kühlraum bei 4 °C gelagert.

Zur Überimpfung wurden 7,5 mL des vorstehend erhaltenen Ansatzes in 500 mL frischem MRS-Medium suspendiert.

Anzucht der Laktobazillen (Variante 2): *Lactobacillus coryniformis* subsp. *coryniformis* (DSM 20001)

Es wird eine ungehopfte Vorderwürze mit 16,5 ° Plato bereitgestellt, welche aus einer Maische mit einer Schüttung von mindestens 50 % Weizenmalz gewonnen wurde. 17 L der Vorderwürze werden jeweils in einen 20 L-Corneliusbehälter eingefüllt, bei 101 °C für etwa 31 min pasteurisiert/autoklaviert und anschließend auf 37 °C abgekühlt.

Das so erhaltene Medium wird mit 255 mL des wie vorstehend erhaltenen *Lactobacillus coryniformis*-Ansatzes angeimpft und bei 37 °C für 48 Stunden fermentiert, woraus ein gebrauchsfertiger *Lactobacillus coryniformis*-Ansatz resultiert.

Anzucht der Laktobazillen: *Lactobacillus rossiae* (DSM 15814) (Variante 1)

Es wurden 500 mL des MRS-Mediums (Fa. Merck, Darmstadt) angesetzt und bei 118 °C für 15 min autoklaviert. Nach dem Abkühlen wurden 5 g Maltose und 5 g Hefeextrakt (Fa. Merck, Darmstadt) zugegeben. Dabei wird das Gefäß randgefüllt, da *Lactobacillus rossiae* mikroaerophil bzw. anaerob ist. Ein Lyophilisat der Lactobazillen wurde im MRS-Medium suspendiert und bei 30 °C für 2 Tage inkubiert. Der so erhaltene Ansatz wurde im Kühlraum bei 4 °C gelagert.

7,5 mL des vorstehend erhaltenen Ansatzes wurden in 500 mL frischem MRS-Medium zur Überimpfung suspendiert.

Anzucht der Laktobazillen: *Lactobacillus rossiae* (DSM 15814) (Variante 2)

Es wird eine ungehopfte Vorderwürze mit 16,5 ° Plato bereitgestellt, welche aus einer Maische mit einer Schüttung von mindestens 50 % Weizenmalz gewonnen wurde. 17 L der Vorderwürze werden jeweils in einen 20 L-Corneliusbehälter eingefüllt, bei 101 °C für etwa 31 min pasteurisiert/autoklaviert und anschließend auf 30 °C abgekühlt.

Das so erhaltene Medium wird mit 255 mL des wie vorstehend beschriebenen *Lactobacillus rossiae*-Ansatzes angeimpft und bei 30 °C für 48 Stunden unter anaeroben Bedingungen fermentiert, woraus ein gebrauchsfertiger *Lactobacillus coryniformis*-Ansatz resultiert. Die anaeroben Bedingungen wurden durch Überschichten des Mediums mit $CO_2$-Gas und Fermentation im geschlossenen Behälter eingestellt.

Erfindungsgemäße Herstellung eines Sauergutes (Variante 1)

In einem 100-mL-Gefäß werden folgende Bestandteile randvoll eingefüllt:
72,4 mL kurzzeiterhitzte Würze, 3 mL der *Lactobacillus rossiae-Anzucht* wie vorstehend beschrieben, 3 mL der *Lactobacillus coryniformis* subsp. *coryniformis*-Anzucht wie vorstehend beschrieben, und 33,6 mL Hefeextrakt wie vorstehend beschrieben.

Dieser Ansatz wird homogenisiert und bei 30 °C für 2 Tage inkubiert (Schritt (b) des Verfahrens), woraus ein Sauergut als erfindungsgemäße Vorstufe eines Nahrungsmittels entsteht.

Erfindungsgemäße Herstellung eines Sauergutes (Variante 2)

Es werden 775,7 L einer ungehopften Vorderwürze mit 16,5 ° Plato bereitgestellt, welche aus einer Maische mit einer Schüttung von mindestens 50 % Weizenmalz gewonnen wurde. Die Vorderwürze wird mit 360 L eines wie vorstehend beschriebenen Hefeextrakts (hergestellt nach vorstehender Variante 2) in einem Fermenter gemischt, bei 80 °C für etwa 10 min pasteurisiert und anschließend auf 37 °C gekühlt. Das Medium wird zur Herstellung von anaeroben Bedingungen mit $CO_2$-Gas überschichtet.

Das so behandelte erste Nährmedium wird mit jeweils einer Anzucht *Lactobacillus coryniformis* subsp. *coryniformis* (DSM 20001) und/oder *Lactobacillus rossiae* (DSM 15814), oder mit einer Kombination aus *Lactobacillus rossiae* (DSM 15814) und *Lactobacillus paracasei* subsp. *paracasei* (DSM 4905); oder aus *Lactobacillus coryniformis* subsp. *coryniformis* (DSM 20001) und *Lactobacillus paracasei* subsp. *paracasei* (DSM 4905), jeweils angezogen gemäß der vorstehend beschriebenen Variante 2), im Volumenverhältnis 1:1, angeimpft. Der Fermenterinhalt wird homogenisiert und bei 37 °C für 2 Tage unter leichtem Rühren inkubiert (Schritt (b) des Verfahrens).

Die Fermentation kann gestoppt werden, wenn der pH-Wert einen Wert von 3,7 erreicht. Zum definierten Stoppen der Fermentation kann der Fermenterinhalt bei 85 °C für 10 Minuten pasteurisiert werden. Des Weiteren kann, wenn gewünscht, das bei der Fermentation entstandene Lactat teilweise oder vollständig durch Mikrofiltration, Dialyse oder ein sonstiges, geeignetes Trennverfahren abgetrennt werden. Hierdurch wird der saure Geschmack des entstandenen Sauerguts reduziert, was je nach Anwendung gewünscht sein kann. Zudem werden tote Zellen entfernt. Anschließend besteht die Möglichkeit, das so erhaltene Sauergut beispielsweise auf 60 ° Brix aufzukonzentrieren, um die Transportmasse und -kosten des so erhaltenen Lebensmittels oder einer Vorstufe desselben zu verringern. Der Konzentrationsschritt kann mittels eines Vakuumverdampfers oder einer anderen geeigneten Verfahrenstechnik bewerkstelligt werden.

Ergebnisse

| Ansatznr. | erstes Nährmedium | Lactobacillus | Hefeerzeugnis | Vitamin $B_{12}$-Konzentration [$\mu$g/100 mL] |
|---|---|---|---|---|
| A1 (Kontrolle) | Würze | - | - | 0,05 |
| A2 | Würze | L. c. | - | 0,58 |
| A3 | Würze | L. r. | - | 0,33 |
| A4 | MRS | L. r. | Hefeextrakt (enthalten in MRS) | 1,53 |
| A5 | Würze | L. r. | Hefeextrakt aus frischer UG Hefe | 0,31 |
| A6 | Würze | L. r. + L. c. | Hefeextrakt aus frischer UG Hefe | 4,52 |
| A7 | Würze | L. r. + L. p. | Hefeextrakt aus frischer UG Hefe | 0,35 |
| A8 | Würze | L. c. + L. p. | Hefeextrakt aus frischer UG Hefe | 0,32 |

L. r. = *Lactobacillus rossiae* (DSM 15814); L. c. = *Lactobacillus coryniformis* subsp. *coryniformis* (DSM 20001); L. p. = *Lactobacillus paracasei* subsp. *paracasei* (DSM 4905); A6, A7, A8: Volumenverhältnis der Lactobazillen: 1:1 bei normierter Zellzahl; UG = untergärig; angegeben ist die Gesamtkonzentration an Vitamin $B_{12}$ im Ansatz, gemessen nach r-Biopharm AOAC-Methode Nr. 101002, wobei die Bioverfügbarkeit des erzeugten Vitamin $B_{12}$ mittels des ADVIA Centaur VB12-Tests bestätigt worden ist.

Wie aus der vorstehenden Tabelle ersichtlich ist, wird durch die erfindungsgemäße Behandlung des ersten Nährmediums mit *Lactobacillus rossiae* eine gegenüber der Kontrolle erhöhte Konzentration an Vitamin $B_{12}$ erhalten (vgl. Ansätze A3 und A5 vs. A1). Eine weitere Steigerung der Vitamin $B_{12}$-Konzentration kann durch Anwesenheit eines Hefeextrakts, wie dies im MRS-Medium enthalten ist, erreicht werden (vgl. Ansatz A4). Eine besonders hohe Konzentration an Vitamin $B_{12}$ wird erhalten, wenn zur Behandlung des Nährmediums eine Kombination aus *Lactobacillus rossiae* und *Lactobacillus coryniformis* eingesetzt wird (vgl. Ansatz A6).

Dagegen wird durch eine Kombination aus *Lactobacillus rossiae* und *Lactobacillus paracasei* eine Konzentration an Vitamin $B_{12}$ erreicht, welche in etwa nur so hoch ist, wie bei der Vergärung mit *Lactobacillus rossiae* alleine (vgl. Ansatz A7 vs. A5). Auch eine Kombination aus *Lactobacillus coryniformis* und *Lactobacillus paracasei* bringt keine Steigerung der Vitamin $B_{12}$-Erzeugung gegenüber der Verwendung von *Lactobacillus coryniformis* alleine (vgl. Ansatz A8 vs. A2). Hieraus wird deutlich, dass sich sehr hohe Konzentrationen an Vitamin $B_{12}$ durch die Kombination von *Lactobacillus rossiae* und *Lactobacillus coryniformis* erreicht wird, nicht jedoch durch eine beliebige Kombination von Lactobacillenarten, auch wenn diese *Lactobacillus rossiae* oder *Lactobacillus coryniformis* enthalten sollte.

| Ansatznr. | erstes Nährmedium | Lactobacillus | Hefeerzeugnis | Vitamin $B_{12}$-Konzentration [$\mu$g/100 mL] |
|---|---|---|---|---|
| B1 (Kontrolle) | Würze | - | - | 0,05 |
| B2 | Würze | L. c. | - | 0,46 |
| B3 | Würze | L. c. | Hefeextrakt aus frischer UG Hefe | 1,29 |

(fortgesetzt)

| Ansatznr. | erstes Nährmedium | Lactobacillus | Hefeerzeugnis | Vitamin $B_{12}$-Konzentration [$\mu$g/100 mL] |
|---|---|---|---|---|
| B4 | Würze | L. c. | Hefeextrakt aus getrockneter UG Hefe | 1,78 |
| B5 | Würze | L. c. + L. r. | Hefeextrakt aus frischer UG Hefe | 4,24 |
| B6 | Würze | L. c. + L. r. | Hefeextrakt aus getrockneter UG Hefe | 5,72 |
| L. r. = *Lactobacillus rossiae* (DSM 15814); L. c. = *Lactobacillus coryniformis* subsp. *coryniformis* (DSM 20001); B5, B6: Volumenverhältnis der Lactobazillen: 1:1 bei normierter Zellzahl; UG = untergärig; angegeben ist die Gesamtkonzentration an Vitamin $B_{12}$ im Ansatz, gemessen nach r-Biopharm AOAC-Methode Nr. 101002, wobei die Bioverfügbarkeit des erzeugten Vitamin $B_{12}$ mittels des ADVIA Centaur VB12-Tests bestätigt worden ist. | | | | |

Wie aus der vorstehenden Tabelle ersichtlich ist, wird durch Zusatz eines Hefeextrakts aus getrockneter gegenüber frischer Hefe eine nochmals verbesserte Ausbeute an Vitamin $B_{12}$ erzielt.

| Ansatznr. | erstes Nährmedium | Lactobacillus | Hefeerzeugnis | Vitamin $B_{12}$-Konzentration [$\mu$g/100 mL] |
|---|---|---|---|---|
| C1 (Kontrolle) | Würze | - | - | 0,05 |
| C2 | Würze | L. r. | - | 0,13 |
| C3 | Würze | L. c. + L. r. | | 0,45 |
| C4 | Würze | L. c. + L. r. | Hefeextrakt aus trockn. UG Hefe, 50 % | 4,93 |
| C5 | Würze | L. c. + L. r. | Hefeextrakt aus trockn. UG Hefe, 40 % | 4,93 |
| C6 | Würze | L. c. + L. r. | Hefeextrakt aus trockn. UG Hefe, 30 % | 5,33 |
| L. r. = *Lactobacillus rossiae* (DSM 15814); L. c. = *Lactobacillus coryniformis* subsp. *coryniformis* (DSM 20001); C3 bis C6: Volumenverhältnis der Lactobazillen: 1:1 bei normierter Zellzahl; UG = untergärig; trockn. = trockener; angegeben ist die Gesamtkonzentration an Vitamin $B_{12}$ im Ansatz, gemessen nach r-Biopharm AOAC-Methode Nr. 101002, wobei die Bioverfügbarkeit des erzeugten Vitamin $B_{12}$ mittels des ADVIA Centaur VB12-Tests bestätigt worden ist. | | | | |

Wie die vorstehende Tabelle zeigt, bestätigt auch dieser Ansatz, dass sich durch die erfindungsgemäße Behandlung von Würze als erstem Nährmedium mit *Lactobacillus rossiae* eine gegenüber der Kontrolle erhöhte Konzentration an Vitamin $B_{12}$ erzielt wird. Eine weitere, deutliche Steigerung der Vitamin $B_{12}$-Konzentration kann durch eine kombinierte Behandlung mit *Lactobacillus rossiae* und *Lactobacillus coryniformis* erzielt werden. Die Ausbeute an Vitamin $B_{12}$ steigt nochmals erheblich durch die Anwesenheit eines Hefeextrakts. Dabei ist ein Anteil an Hefeextrakt von 50 % nicht erforderlich, mit einem Volumenanteil von 30 % wurden in dieser Versuchsreihe die höchsten Vitamin-Konzentrationen erzielt.

Verkostungsuntersuchungen zeigten, dass Biere, welche mit einem Sauergut, hergestellt nach dem erfindungsgemäßen Verfahren, hergestellt wurden, einen Geruch aufwiesen, der an Brot bzw. Sauerteigbrot erinnerte. Dies war vor allem bei Verwendung von Hefeextrakt aus frischer Hefe der Fall. Im Falle der Verwendung eines Zusatzes aus Trockenhefe war die Intensität des Sauerteigbrots erheblich geringer. Ein analoger Eindruck war in Bezug auf die geschmackliche Beurteilung der Biere festzustellen.

In einem weiteren Ansatz sollte herausgefunden werden, mit welcher Menge an Hefeextrakt sich die höchste Ausbeute an bioverfügbarem Vitamin $B_{12}$ erzielen lässt. Hierzu wurden die zugesetzten Mengen an Hefeextrakt bei ansonsten gleichen Bedingungen variiert.

| Ansatznr. | Hefeerzeugnis (vol.% bezogen auf das Volumen des ersten Nährmediums) | Hefeerzeugnis (g/L bezogen auf das Volumen des ersten Nährmediums) | Vitamin $B_{12}$-Konzentration [$\mu$g/ 100 mL] |
|---|---|---|---|
| D1 (Kontrolle) | 0 | 0 | 0,62 |
| D2 | 5 | 3,8 | 1,92 |
| D3 | 10 | 7,9 | 2,92 |
| D4 | 20 | 17,9 | 4,48 |
| D5 | 30 | 30,6 | 4,88 |
| D6 | 40 | 47,6 | 3,14 |
| D7 | 50 | 71,4 | 2,46 |

eingesetztes erstes Nährmedium: Würze; eingesetzte Lactobcilli: Mischung aus *Lactobacillus rossiae* (DSM 15814) und *Lactobacillus coryniformis* subsp. *coryniformis* (DSM 20001); jeweils im Volumenverhältnis 1:1 bei normierter Zellzahl; Hefeerzeugnis: Hefeextrakt aus untergäriger Trockenhefe, hergestellt nach vorstehend beschriebener Variante 1; angegeben ist die Gesamtkonzentration an Vitamin $B_{12}$ im Ansatz, gemessen nach r-Biopharm AOAC-Methode Nr. 101002, wobei die Bioverfügbarkeit des erzeugten Vitamin $B_{12}$ mittels des ADVIA Centaur VB12-Tests bestätigt worden ist.

Wie die vorstehende Tabelle zeigt, wurde bei einer Zugabe eines Hefeextrakts bereits in einer Menge von 3,8 g Hefeextrakt auf 1 L erstem Nährmedium (entspricht 5 vol.% eingesetztes Hefeextrakt bezogen auf das erste Nährmedium) eine deutliche Steigerung der Konzentration an bioverfügbarem Vitamin $B_{12}$ gemessen. Bei einem Zusatz von ca. 30 g Hefeextrakt auf 1 L erstem Nährmedium (entspricht 30 vol.% eingesetztes Hefeextrakt bezogen auf das erste Nährmedium) wurde mit fast 5 $\mu$g/100 mL die höchste Konzentration gemessen. Wird die Hefeextraktzugabe weiter bis ca. 70 g/L gesteigert, werden hohe Konzentrationen an bioverfügbarem Vitamin $B_{12}$ im Nährmedium erzielt. Die erzeugten Mengen nehmen jedoch wieder ab. Nach diesem Ansatz erscheint eine Zugabemenge an Hefeerzeugnis zwischen 4 und 70 g/L, vorzugsweise zwischen 8 und 50 g/L, vorzugsweise zwischen 12 und 40 g/L, insbesondere zwischen 18 und 35 g/L, bezogen auf das Volumen des ersten Nährmediums, eine besonders hohe Ausbeute zu ergeben.

In einem weiteren Ansatz sollte herausgefunden werden, wie sich die Inkubationstemperatur der Hefe während der Gewinnung des Hefeerzeugnisses auf die Erzeugung an bioverfügbarem Vitamin $B_{12}$ nach dem erfindungsgemäßen Verfahren auswirkt. Hierzu wurde als Hefeerzeugnis ein Hefeextrakt aus untergäriger Trockenhefe nach der vorstehend beschriebenen Variante 1 hergestellt, wobei die Inkubation einmal bei 57 °C (Standardverfahren) und in der Variante bei 80 °C durchgeführt wurde. Zusätzlich wurde die Konzentration des Hefeerzeugnisses variiert. Alle anderen Bedingungen waren gleich.

| Ansatznr. | Hefeerzeugnis [g/L; bezogen auf das Volumen des ersten Nährmediums] | Inkubationstemperatur [°C] | Vitamin $B_{12}$-Konzentration [$\mu$g/100 mL] |
|---|---|---|---|
| E1 (Kontrolle) | 0 | 57 | 0,52 |
| E2 | 17,9 | 57 | 3,84 |
| E3 | 35,7 | 57 | 5,60 |
| E4 | 35,7 | 80 | 0,20 |
| E5 | 71,4 | 57 | 8,96 |
| E6 | 71,4 | 80 | 0,32 |

eingesetztes erstes Nährmedium: Würze; eingesetzte Lactobcilli: Mischung aus *Lactobacillus rossiae* (DSM 15814) und *Lactobacillus coryniformis* subsp. *coryniformis* (DSM 20001); jeweils im Volumenverhältnis 1:3 bei normierter Zellzahl; Hefeerzeugnis: Hefeextrakt aus untergäriger Trockenhefe, hergestellt nach vorstehend beschriebener Variante 1 mit Inkubationstemperatur 57 oder 80 °C; angegeben ist die Gesamtkonzentration an Vitamin $B_{12}$ im Ansatz, gemessen nach r-Biopharm AOAC-Methode Nr. 101002, wobei die Bioverfügbarkeit des erzeugten Vitamin $B_{12}$ mittels des ADVIA Centaur VB12-Tests bestätigt worden ist.

Wie die vorstehende Tabelle zeigt, wurde bei dieser Untersuchung eine zunehmende Konzentration an bioverfügbarem Vitamin $B_{12}$ mit zunehmender Menge an Hefeerzeugnis (Inkubation bei 57 °C) beobachtet. Wurde jedoch bei ansonsten gleichen Bedingungen die Inkubationstemperatur von 57 °C auf 80 °C bei der Herstellung des Hefeextrakts erhöht, resultierte eine erheblich geringere Vitamin $B_{12}$-Konzentration.

Variation der Mischungsverhältnisse der Lactobacilli

In einer weiteren Untersuchungsreihe wurde untersucht, ob sich die Ausbeute an Vitamin $B_{12}$ weiter steigern lässt, wenn die beiden verwendeten Milchsäurebakterien *Lactobacillus rossiae* und *Lactobacillus coryniformis* subsp. *coryniformis* in unterschiedlichen Mischungsverhältnissen eingesetzt werden.

Bei allen Ansätzen wurden 72 mL Würze mit 33 mL Trockenhefe und 6 mL Baktereinsuspension (in MRS) gemischt, wobei die Bakteriensuspension jeweils die folgende Zusammensetzung aufwies:

Ansatz F2:    1,5 mL *L. coryniformis* + 4,5 mL *L. rossiae*

Ansatz F3:    3,0 mL *L. coryniformis* + 3,0 mL *L. rossiae*

Ansatz F4:    4,5 mL *L. coryniformis* + 1,5 mL *L. rossiae*

Der jeweilige Ansatz wurde bei 37°C im Brutschrank für 48 h inkubiert. Anschließend wurden die Ansätze im Wasserbad für 30 min auf 95 °C erwärmt. Bis zur Vitamin $B_{12}$-Bestimmung wurden die Proben eingefroren.

Die Vitamin $B_{12}$-Gehalte wurden mikrobiologisch bestimmt:

$$20 \text{ mL Probe} + 20 \text{ mL (Na-Acetat pH 4,5} + 1 \text{ \% KCN} + \alpha\text{-Amylase, Pepsin)}.$$

Die weitere Durchführung erfolgte gemäß der Methode r-Biopharm AOAC-Methode Nr. 101002.

| Ansatznr. | erstes Nährmedium | Lactobacilli (Volumenverhältnis L. c. : L. r.) | Vitamin $B_{12}$-Konzentration [$\mu$g/100 mL] |
|---|---|---|---|
| F1 (Kontrolle) | Würze | - | 0,05 |
| F2 | Würze | 1 : 3 | 1,60 |
| F3 | Würze | 1 :1 | 2,54 |
| F4 | Würze | 3 : 1 | 2,86 |

Angegeben ist die Gesamtkonzentration an Vitamin $B_{12}$ im Ansatz, gemessen nach r-Biopharm AOAC-Methode Nr. 101002, wobei die Bioverfügbarkeit des erzeugten Vitamin $B_{12}$ mittels des ADVIA Centaur VB12-Tests bestätigt worden ist.

Wie aus der vorstehend Tabelle zu entnehmen ist, kann eine Zunahme der Vitamin $B_{12}$-Synthese bei einem steigenden *L. coryniformis*-Anteil im Inoculum erzielt werden. Die absoluten Konzentrationen sind mit den vorstehenden Versuchsergebnissen aufgrund des unterschiedlichen Zustandes der eingesetzten Lacotbacillen nur beschränkt vergleichbar.

Folglich sollte der *L. coryniformis*-Anteil im eingesetzten Lacotbacillen-Gemisch wenigstens 50 vol.-%, vorzugsweise 60 bis 90 vol.-%, betragen.

Vergleich zum Stand der Technik

Herkömmlicherweise werden für die Maische- bzw. Würzesäuerung die Arten *Lactobacillus amylovorus* bzw. *Lactobacillus amylolyticus* verwendet, welche sich zur Erzeugung von entsprechenden Sauergüten und damit angesäuerten Maischen und Würzen langjährig bewährt haben.

So zeichnen sich diese Milchsäurearten durch eine Wachstumsdominanz in Bierwürze wegen Schnellwüchsigkeit aus. Ferner weisen sie ein hohes Säuerungsvermögen aufgrund einer hohen Laktat-Produktion auf. Dies ist auf ihren homofermentativen Stoffwechselcharakter zurückzuführen. Diese Arten wachsen bei hohen Temperaturen (bis 52 °C), so dass sich hohe Vermehrungsraten erzielen lassen.

Darüber hinaus können diese Arten Dextrin und Stärke vergären. Ferner erzeugen sie einen hohen Anteil an L(+)-Laktat. Von erheblicher Bedeutung ist, dass die genannten Milchsäurebakterien nicht bierschädlich sind, da sie hopfenempfindlich sind und bei Temperaturen < 30 °C nicht wachsen können. In der Fachwelt werden *Lactobacillus amylovorus* und *Lactobacillus amylolyticus* auch deshalb als geeignete Säuerungsorganismen angesehen, weil sie keine Amine (Histamin) oder andere Toxine ausbilden. Darüber hinaus bilden sie kein Diacetyl oder andere, für Geschmack und Aroma der resultierenden Erzeugnisse nachteiligen Substanzen. Schließlich zeichnen sie sich durch eine leichte Handhabbarkeit in der praktischen Verwendung aus.

Demgegenüber gilt die erfindungsgemäß vorgesehene Art *Lactobacillus rossiae,* die erfindungsgemäß vorzugsweise in Kombination mit *Lactobacillus coryniformis* eingesetzt wird, in der Fachwelt als Bierschädling. Dies gilt ebenso für die Art *Lactobacillus coryniformis. Lactobacillus rossiae* ist dem Fachmann in der Brauerei als Schleimbildner bekannt. Beide Arten wachsen in schwach gehopftem Bier und erzeugen Diacetyl, welches zu einem nachteiligen Geschmacksprofil im resultierenden Nahrungsmittel oder Getränk, insbesondere im Bier, führt. Darüber hinaus sind sie in der Lage, bei den typischen Vergärungstemperaturen für Bier, insbesondere für obergäriges Bier, nämlich im Temperaturbereich von 15 bis 48 °C, zu wachsen. Weiterhin weisen diese Arten gegenüber den herkömmlichen zur Säuerung eingesetzten Arten *Lactobacillus amylovorus* und *Lactobacillus amylolyticus* den Nachteil auf, dass diese Art fakultativ heterofermentativ ist, d.h., deren Säuerungsvermögen ist gegenüber den herkömmlich verwendeten Arten vermindert. Folglich muss in etwa die doppelte Menge an Sauergut im Vergleich zu herkömmlichen Arten eingesetzt werden. Hierdurch werden größere Produktionsanlagen erforderlich, und die mit der Säuerung verbundenen Kosten steigen.

Die vorstehend genannte Mehrzahl von Nachteilen sowie die dem Fachmann darüber hinaus bekannten Nachteile der hier betrachteten Arten *Lactobacillus rossiae* und *Lactobacillus coryniformis* bedeuteten bisher ein wesentliches Hindernis für den Einsatz dieser Arten oder zugehöriger Unterarten in der Getränke- und Nahrungsmittelherstellenden Industrie, insbesondere in Brauereien und Mälzereien.

### Patentansprüche

1. Verfahren zur Herstellung eines Nahrungsmittels oder einer Vorstufe desselben, wenigstens mit den Schritten:

   (a) Bereitstellen einer Maische oder einer Würze oder eines Glattwassers als ein erstes Nährmedium; und
   (b) Behandeln des ersten Nährmediums mit Milchsäurebakterien der Art *Lactobacillus rossiae* (DSM 15814$^T$) oder mit Milchsäurebakterien wenigstens umfassend zwei Arten, darunter *Lactobacillus rossiae* (DSM 15814$^T$).

2. Verfahren nach Anspruch 1, **dadurch gekennzeichnet, dass** das Verfahren den Schritt aufweist:

   (b) Behandeln des ersten Nährmediums mit Milchsäurebakterien der Arten *Lactobacillus rossiae* (DSM 15814$^T$) und *Lactobacillus coryniformis,* insbesondere der Unterart *Lactobacillus coryniformis* subsp. *coryniformis* (DSMZ Nr. 20007), vorzugsweise mit einem Gemisch enthaltend Milchsäurebakterien der Arten *Lactobacillus rossiae* (DSM 15814$^T$) und *Lactobacillus coryniformis,* insbesondere der Unterart *Lactobacillus coryniformis* subsp. *coryniformis* (DSMZ Nr. 20007).

3. Verfahren nach Anspruch 1 oder 2, **dadurch gekennzeichnet, dass** die Behandlung gemäß Schritt (b) in Anwesenheit eines Hefeerzeugnisses stattfindet;
   wobei das Hefeerzeugnis vorzugsweise ein Extrakt, ein Autolysat und/oder eine Hefe in frischer oder getrockneter Gestalt enthält oder daraus besteht; und/oder
   wobei das Hefeerzeugnis vorzugsweise in einer Massenkonzentration im Bereich von ≥ 0,2 bis ≤ 70 g/L, vorzugsweise von ≥ 8 bis ≤ 50 g/L, bezogen auf das erste Nährmedium vorliegt.

4. Verfahren nach einem der Ansprüche 1 bis 3, **dadurch gekennzeichnet, dass** das erste Nährmedium frei oder im Wesentlichen frei von Hopfenbitterstoffen ist; und/oder
   das Hefeerzeugnis frei oder im Wesentlichen frei von Hopfenbitterstoffen ist.

5. Verfahren nach einem der Ansprüche 1 bis 4, **dadurch gekennzeichnet, dass** das Hefeerzeugnis aus einer obergärigen oder untergärigen Hefe der Gattung *Saccharomyces,* insbesondere der Art *Saccharomyces cerevisiae* oder der Art *Saccharomyces carlsbergensis,* gewonnen wird;
   wobei die Hefe vorzugsweise eine Reinzuchthefe oder eine Erntehefe aus dem Bierbereitungsverfahren ist; und/oder
   wobei Hopfenbitterstoffe aus der Hefe oder dem Hefeerzeugnis vollständig oder im Wesentlichen vollständig entfernt werden, vorzugsweise durch ein- oder mehrmaliges Waschen der Hefe oder des Hefeerzeugnisses mit Wasser, insbesondere mit Trinkwasser oder Brauwasser.

6. Verfahren nach einem der Ansprüche 1 bis 5, **dadurch gekennzeichnet, dass** das Verfahren ferner die Schritte aufweist:

(c) Bereitstellen einer Maische oder einer Würze als ein zweites Nährmedium; und
(d) Mischen des in Schritt (b) erhaltenen Mediums mit dem zweiten Nährmedium.

**7.** Verfahren nach einem der Ansprüche 1 bis 6, **dadurch gekennzeichnet, dass** das Verfahren ferner die Schritte aufweist:

(e) vorzugsweise Läutern des in Schritt (b) oder (d) erhaltenen Mediums;
(f) Kochen oder Heißhalten und vorzugsweise Hopfung des in Schritt (d) oder (e) erhaltenen Mediums;
(g) wenigstens teilweises Entfernen von Trub aus dem in Schritt (f) erhaltenen Medium; und
(h) vorzugsweise Einstellen der Temperatur des in Schritt (g) erhaltenen Mediums auf eine Anstelltemperatur.

**8.** Verfahren nach einem der Ansprüche 1 bis 7, **dadurch gekennzeichnet, dass** das Verfahren ferner wenigstens einen der Schritte aufweist:

(i) Verarbeiten des in einem der Schritte (b), (d), (f), (g) oder (h) erhaltenen Mediums zu einem Getränk, vorzugsweise Behandeln dieses Mediums mit einer Hefe der Gattung *Saccharomyces;* und/oder
(k) Mischen des in einem der Schritte (b), (d), (f), (g) oder (h) erhaltenen Mediums mit einem Getränk, vorzugsweise mit einem Bier.

**9.** Verfahren nach einem der Ansprüche 1 bis 8, **dadurch gekennzeichnet, dass** das Verfahren ferner den Schritt aufweist:

(1) Verarbeiten des in einem der Schritte (b), (d), (f), (g), oder (h) erhaltenen Mediums oder des in Schritt (i) oder (k) erhaltenen Getränks zu einem nicht fluiden Nahrungsmittel;

wobei das in einem der Schritte (b), (d), (f), (g) oder (h) erhaltene Medium oder das in Schritt (i) oder (k) erhaltene Getränk mit einer Vorstufe des nicht fluiden Nahrungsmittels gemischt wird.

**10.** Verfahren nach einem der Ansprüche 1 bis 9, **dadurch gekennzeichnet, dass** das Verfahren ferner den Schritt aufweist:

(t) Einstellen des Massenanteils an Wasser im Medium, welches in einem der Schritte (b), (d), (f), (g) oder (h) erhalten wurde, im in Schritt (i) oder (k) erhaltenen Getränk oder im in Schritt (1) erhaltenen, nicht fluiden Nahrungsmittel auf weniger als 35 %, vorzugsweise weniger als 30 %, vorzugsweise weniger als 25 %, vorzugsweise auf weniger als 20 %, insbesondere auf weniger als 15 %; und vorzugsweise auf mehr als 0 %, insbesondere mehr als 5 %.

**11.** Nahrungsmittel oder eine Vorstufe desselben, hergestellt mit einem Verfahren nach einem der Ansprüche 1 bis 10.

**12.** Verwendung von Milchsäurebakterien der Art *Lactobacillus rossiae* (DSM 15814$^T$) oder von Milchsäurebakterien wenigstens umfassend zwei Arten, darunter *Lactobacillus rossiae* (DSM 15814$^T$), vorzugsweise eines Gemisches aus Milchsäurebakterien der Arten *Lactobacillus rossiae* (DSM 15814$^T$) und *Lactobacillus coryniformis,* insbesondere der Unterart *Lactobacillus coryniformis* subsp. *coryniformis* (DSMZ Nr. 20007), in einem Verfahren zur Herstellung eines Nahrungsmittels oder einer Vorstufe desselben, vorzugsweise in einem Verfahren nach einem der Ansprüche 1 bis 10,
wobei das Verfahren wenigstens die Schritte aufweist:

(a) Bereitstellen einer Maische oder einer Würze oder eines Glattwassers als ein erstes Nährmedium; und
(b) Behandeln des ersten Nährmediums mit Milchsäurebakterien der Art *Lactobacillus rossiae* (DSM 15814$^T$) oder mit Milchsäurebakterien wenigstens umfassend zwei Arten, darunter *Lactobacillus rossiae* (DSM 15814$^T$), vorzugsweise mit einem Gemisch enthaltend Milchsäurebakterien der Arten *Lactobacillus rossiae* (DSM 15814$^T$) und *Lactobacillus coryniformis,* insbesondere der Unterart *Lactobacillus coryniformis* subsp. *coryniformis* (DSMZ Nr. 20007).

wobei die Behandlung des Schrittes (b) vorzugsweise in Anwesenheit des Hefeerzeugnisses stattfindet; und
wobei das Hefeerzeugnis vorzugsweise ein Extrakt, ein Autolysat und/oder eine Hefe in frischer oder getrockneter Gestalt enthält.

13. Verwendung nach Anspruch 12, **dadurch gekennzeichnet, dass** das Hefeerzeugnis in einer Massenkonzentration im Bereich von ≥ 0,2 bis ≤ 70 g/L, vorzugsweise von ≥ 8 bis ≤ 50 g/L, bezogen auf das erste Nährmedium vorliegt.

14. Verwendung nach Anspruch 12 oder 13, **dadurch gekennzeichnet, dass** das erste Nährmedium frei oder im Wesentlichen frei von Hopfenbitterstoffen ist; und/oder das Hefeerzeugnis frei oder im Wesentlichen frei von Hopfenbitterstoffen ist.

**Europäisches Patentamt**
**European Patent Office**
**Office européen des brevets**

## EUROPÄISCHER RECHERCHENBERICHT

Nummer der Anmeldung

EP 18 19 0459

### EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | MARIA DE ANGELIS ET AL: "Lactobacillus rossiae, a Vitamin B12 Producer, Represents a Metabolically Versatile Species within the Genus Lactobacillus", PLOS ONE, Bd. 9, Nr. 9, 29. September 2014 (2014-09-29), Seite e107232, XP055486733, DOI: 10.1371/journal.pone.0107232 | 1,3, 11-13 | INV. C12C5/02 C12C12/00 A23L31/15 |
| Y | * Seite 10, Absatz 3 * * Zusammenfassung * ----- | 1-14 | |
| X | WO 2008/010252 A2 (GIULIANI SPA [IT]; GIULIANI GIAMMARIA [IT]; BENEDUSI ANNA [IT]; DI CAG) 24. Januar 2008 (2008-01-24) * Ansprüche 1-14; Beispiel 1 * ----- | 1,3-6, 9-14 | |
| Y | EP 2 995 204 A1 (ERDINGER WEISSBRÄU WERNER BROMBACH GMBH & CO KG [DE]) 16. März 2016 (2016-03-16) * Ausführungsbeispiele; Ansprüche 1-13 * ----- | 1-14 | |

RECHERCHIERTE SACHGEBIETE (IPC)

C12C
A23L

Der vorliegende Recherchenbericht wurde für alle Patentansprüche erstellt

| Recherchenort | Abschlußdatum der Recherche | Prüfer |
|---|---|---|
| Den Haag | 4. Januar 2019 | Granet, Nicolas |

KATEGORIE DER GENANNTEN DOKUMENTE

X : von besonderer Bedeutung allein betrachtet
Y : von besonderer Bedeutung in Verbindung mit einer
  anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
  nach dem Anmeldedatum veröffentlicht worden ist
D : in der Anmeldung angeführtes Dokument
L : aus anderen Gründen angeführtes Dokument

................................................................

& : Mitglied der gleichen Patentfamilie, übereinstimmendes
  Dokument

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 18 19 0459

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

04-01-2019

| Im Recherchenbericht angeführtes Patentdokument | Datum der Veröffentlichung | Mitglied(er) der Patentfamilie | Datum der Veröffentlichung |
|---|---|---|---|
| WO 2008010252 A2 | 24-01-2008 | AU 2007274602 A1 | 24-01-2008 |
| | | CA 2657899 A1 | 24-01-2008 |
| | | CN 101495617 A | 29-07-2009 |
| | | CY 1113397 T1 | 22-06-2016 |
| | | DK 2046944 T3 | 07-01-2013 |
| | | EP 2046944 A2 | 15-04-2009 |
| | | ES 2395391 T3 | 12-02-2013 |
| | | JP 5289311 B2 | 11-09-2013 |
| | | JP 2009543576 A | 10-12-2009 |
| | | PT 2046944 E | 24-12-2012 |
| | | SI 2046944 T1 | 31-01-2013 |
| | | SM T201300009 B | 06-05-2013 |
| | | US 2010055238 A1 | 04-03-2010 |
| | | US 2015216185 A1 | 06-08-2015 |
| | | WO 2008010252 A2 | 24-01-2008 |
| EP 2995204 A1 | 16-03-2016 | DE 102014110182 A1 | 21-01-2016 |
| | | DK 2995204 T3 | 10-04-2017 |
| | | EP 2995204 A1 | 16-03-2016 |
| | | ES 2622115 T3 | 05-07-2017 |

EPO FORM P0461

Für nähere Einzelheiten zu diesem Anhang : siehe Amtsblatt des Europäischen Patentamts, Nr.12/82

**EP 3 450 534 A1**

**IN DER BESCHREIBUNG AUFGEFÜHRTE DOKUMENTE**

**In der Beschreibung aufgeführte Patentdokumente**

- WO 2012109324 A1
- EP 824152 B1
- GB 793467 A